(19) Europäisches Patentamt
European Patent Office
Office européen des brevets

(11) EP 3 978 518 A1

(12) EUROPEAN PATENT APPLICATION
published in accordance with Art. 153(4) EPC

(43) Date of publication:
**06.04.2022 Bulletin 2022/14**

(21) Application number: **20793990.1**

(22) Date of filing: **22.04.2020**

(51) International Patent Classification (IPC):
***C07K 14/475*** (2006.01) ***A61K 38/18*** (2006.01)
***A61K 47/68*** (2017.01) ***A61P 1/16*** (2006.01)
***A61P 3/04*** (2006.01) ***A61P 3/10*** (2006.01)

(52) Cooperative Patent Classification (CPC):
**A61K 38/18; A61K 47/68; A61P 1/16; A61P 3/04; A61P 3/10; C07K 14/475**

(86) International application number:
**PCT/KR2020/005324**

(87) International publication number:
**WO 2020/218827 (29.10.2020 Gazette 2020/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.04.2019 KR 20190047558**

(71) Applicant: **LG CHEM, LTD.**
**Yeongdeungpo-gu,**
**Seoul 07336 (KR)**

(72) Inventors:
- **KIM, Yeonchul**
**Daejeon 34122 (KR)**
- **MIN, Kyeongsik**
**Daejeon 34122 (KR)**
- **SON, Young Dok**
**Daejeon 34122 (KR)**
- **NA, Kyubong**
**Daejeon 34122 (KR)**
- **HONG, Ji Ho**
**Daejeon 34122 (KR)**
- **JUNG, Saem**
**Daejeon 34122 (KR)**
- **JIN, Myung Won**
**Daejeon 34122 (KR)**
- **PARK, Ji A**
**Daejeon 34122 (KR)**
- **NOH, Soomin**
**Daejeon 34122 (KR)**

(74) Representative: **Hoffmann Eitle**
**Patent- und Rechtsanwälte PartmbB**
**Arabellastraße 30**
**81925 München (DE)**

# (54) FUSION POLYPEPTIDE COMPRISING FC REGION OF IMMUNOGLOBULIN AND GDF15

(57) Provided is a fusion polypeptide comprising GDF15 (Growth/differentiation factor 15) and an Fc region of immunoglobulin, a pharmaceutical composition comprising the fusion polypeptide, and a method of increasing in vivo duration of GDF15 comprising fusing with an Fc region of immunoglobulin.

【FIG. 7】

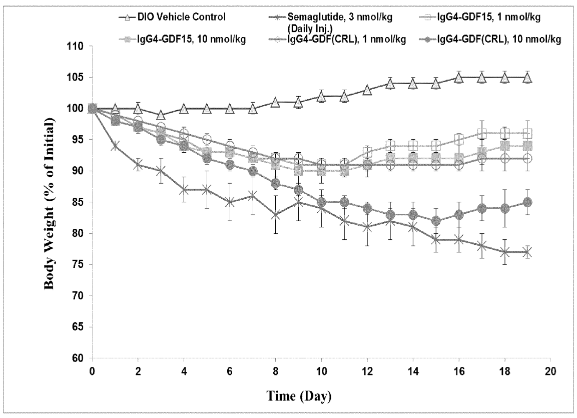


EP 3 978 518 A1

## Description

[TECHNICAL FIELD]

Provided is a fusion polypeptide comprising GDF15 (Growth/differentiation factor 15) and an Fc region of immunoglobulin, a pharmaceutical composition comprising the fusion polypeptide, and a method of increasing in vivo duration of GDF15 comprising fusing with an Fc region of immunoglobulin.

[BACKGROUND ART]

Most protein or peptide drugs have a short period of activity in the body and have a low absorption rate when administered by a method other than intravenous administration, and therefore they have the inconvenience of continuously injecting these drugs repeatedly at short administration intervals when treatment with long-term medication is required. To solve this inconvenience, it is required to develop a technique for continuously releasing drugs in a single dose. As a response to such needs, sustained release formulations for sustained release are being developed.
For example, research for sustained release formulations in which microparticles in a form of surrounding a protein or peptide drug by a biodegradable polymer matrix are prepared and during administration of them, the matrix material is slowly decomposed and is removed in the body and the drug is slowly released has been actively conducted.
On the other hand, GDF15 (Growth/differentiation factor 15) is one of TGF-beta family, and is a 25kDa homodimer, and is a secretory protein circulating in plasma. The plasma level of GDF15 is related to BMI (body mass index) and GDF15 plays a role as a long-term regulator of energy homeostasis. GDF15 also protects against pathological conditions such as cardiovascular disease, myocardial hypertrophy, ischemic damage, and the like. In addition, GDF15 protects from renal tubular and renal interstitial damage in type 1 diabetes and type 2 diabetes models. Furthermore, GDF15 has a protective effect against age-related sensory and motor nerve loss and may contribute to the recovery of peripheral nerve damage. Moreover, GDF15 has weight loss and body fat reduction and glucose resistance effects, and has an effect of increasing systemic energy consumption and oxidative metabolism. GDF15 exhibits a glycemic control effect through weight-dependent and non-dependent mechanisms.
There is a need to develop a technique for improving the persistence of GDF15 protein in the body having various pharmacological effects.

[DISCLOSURE]

[TECHNICAL PROBLEM]

In this disclosure, provided is a technology of constructing a fusion polypeptide by linking GDF15 (Growth/differentiation factor 15) or its functional variant with an Fc region of immunoglobulin; thereby enhancing in vivo duration by increasing in vivo half-life of GDF15, compared to the case of being free of an Fc region, to enhance the pharmacological effect of GDF15 and/or increase the administration interval.
An embodiment provides a fusion polypeptide comprising GDF15 or its functional variant and an Fc region of immunoglobulin.
In the fusion polypeptide, the Fc region of immunoglobulin may be comprised (linked) in the N-terminus of the GDF15 or its functional variant. For example, the fusion polypeptide may comprise (1) an Fc region of immunoglobulin and (2) GDF15 or its functional variant from the N-terminus to the C-terminus.
The Fc region of immunoglobulin comprised in the fusion polypeptide plays a role of enhancing the stability of GDF15 or its variant, and for example, it may have an effect of extending the half-life (e.g., in vivo half-life). In one embodiment, the Fc region of immunoglobulin may be selected from the group consisting of an Fc region of IgG1 and an Fc region of IgG4. The Fc region of IgG1 may comprise CH2 domain, CH3 domain, or CH2+CH3 domain of IgG1, and comprise or be free of a hinge region of IgG1 at the N-terminus. The Fc region of IgG4 may comprise CH2 domain, CH3 domain, or CH2+CH3 domain of IgG4, and comprise or be free of a hinge region of IgG4 at the N-terminus.
The fusion polypeptide may further comprise a peptide linker between the Fc region of immunoglobulin and GDF15 or its functional variant. In one embodiment, the peptide linker may be a flexible linker, so that the Fc region and GDF15 which are fused to both end of the linker can be independently exhibit its function. In a specific embodiment, the peptide linker may not be a rigid linker. For example, the peptide linker may be a GS linker which repeatedly comprises at least one Gly (G) and at least one Ser (S), and for example, may be represented by (GGGGS)n (n is the repeat number of GGGGS (SEQ ID NO: 13), and is an integer from 1 to 10, or an integer from 1 to 5 (1, 2, 3, 4, or 5)), but not limited thereto.
In the fusion polypeptide, the GDF15 or its functional variant fused with the Fc region of immunoglobulin is characterized by increased in vivo (or in blood) stability (duration), compared to GDF15 or its functional variant not fused with an Fc region of immunoglobulin (for example, in vivo or in blood half-life is increased). In addition, the GDF15 or its functional variant fused with the Fc region of immunoglobulin is characterized by the improved pharmacological effect (for example. weight loss effect), compared to GDF15 or its functional variant not fused with an Fc region of immunoglobulin.

Other embodiment provides a fusion polypeptide dimer comprising 2 of the fusion polypeptides. The fusion polypeptide dimer may be formed by linking (combining) to the GDF15 or its functional variant of 2 of the fusion polypeptides.

Other embodiment provides a nucleic acid molecule encoding the fusion polypeptide. Other embodiment provides a recombinant vector comprising the nucleic acid molecule. Other embodiment provides a recombinant cell comprising the recombinant vector.

Other embodiment provides a composition (pharmaceutical composition or health functional food composition) for weight loss, dietary control (reducing the size of meals), or prevention, improvement, alleviation, and/or treatment of metabolic disease, comprising at least one selected from the group consisting of the fusion polypeptide, fusion polypeptide dimer, nucleic acid molecule encoding the fusion polypeptide, recombinant vector comprising the nucleic acid molecule, and the recombinant cell comprising the recombinant vector. Other embodiment provides a method of weight loss, method for dietary control (reducing the amount of meals), or a method of prevention, improvement, alleviation, and/or treatment of metabolic disease, comprising administering a pharmaceutically effective dose of at least one selected from the group consisting of the fusion polypeptide, fusion polypeptide dimer, nucleic acid molecule encoding the fusion polypeptide, recombinant vector comprising the nucleic acid molecule, and the recombinant cell comprising the recombinant vector, to a subject in need of prevention, improvement, alleviation and/or treatment of metabolic disease. The metabolic disease means all diseases caused by metabolic disorder, and it may be selected from the group consisting of obesity, diabetes (for example, type 2 diabetes), nonalcoholic fatty liver disease (for example, nonalcoholic steatohepatitis (NASH), etc.), and the like.

Other embodiment provides a method of preparation of GDF15 or its functional variant with increased in vivo (or in blood) half-life, comprising expressing the recombinant vector in a cell, or a method for preparation of a fusion polypeptide comprising GDF15 or its functional variant with increased in vivo (or in blood) half-life or a homodimer comprising the fusion polypeptide.

Other embodiment provides a method of increasing in vivo duration of GDF15 or its functional variant comprising fusing (or linking or combining) GDF15 or its functional variant with an Fc region of immunoglobulin. In one specific embodiment, the fusing may comprise fusing (or linking or combining) an Fc region of immunoglobulin to an N-terminus of GDF15 or its functional variant with or without a linker. Another embodiment provides a method of decreasing immunogenicity of GDF15, an Fc region of immunoglobulin, or a fusion polypeptide comprising both of them, comprising fusing (or linking or combining) an Fc region of immunoglobulin to GDF15 or its functional variant with a flexible linker (for example, GS linker). The step of fusing (or linking or combining) may be conducted in vitro.

[TECHNICAL SOLUTION]

Hereinafter, the present invention will be described in more detail:

<u>GDF15</u>

GDF15 consists of amino acids from 197th (A) to 308th (I), except for a signal peptide and propeptide in the 308 amino acids in total (UniProt Q99988) (SEQ ID NO: 1; FIG. 2; mature form):

Herein, GDF15 means a polypeptide essentially comprising the amino acid sequence from 197th (A) to 308th (I) of the full-length protein (UniProt Q99988) (SEQ ID NO: 1, See FIG. 2; ARNG DHCPLGPGRC CRLHTVRASL EDLGWADWVL SPREVQVTMC IGACPSQFRA ANMHAQIKTS LHRLKPDTVP APCCVPASYN PMVLIQKTDT GVSLQTYDDL LAKDCHCI), or an amino acid sequence having a sequence homology of 80% or higher, 85% or higher, 90% or higher, 95% or higher, 96% or higher, 97% or higher, 98% or higher, or 99% or higher with the amino acid sequence in the range that maintains the unique activity and structure of GDF15, unless mentioned otherwise.

Herein, the functional variant of GDF15 may be a variant modified so that GDF15 maintains the unique activity and structure and is also advantageous for dimer structure formation. In one embodiment, the functional variant of GDF15 may be an N-terminus deletion variant in which at least one (1, 2, 3, 4, 5, 6, 7, 8, 9, 10, 11, 12, 13, or 14) of 14 amino acid residues of the N-terminus of the amino acid sequence of GDF15 of SEQ ID NO: 1 (namely, 14 amino acid residues in total from 1st to 14th in SEQ ID NO: 1) (for example, at least one from the N-terminus in order), for example, all 14 amino acid residues are deleted (hereinafter, may be represented by '∆N14GDF15' or 'GDF(CRL)'). In one specific embodiment, the functional variant of GDF15 may be a polypeptide essentially comprising the amino acid sequence of SEQ ID NO: 2 (CRLHTVRASL EDLGWADWVL SPREVQVTMC IGACPSQFRA ANMHAQIKTS LHRLKPDTVP APCCVPASYN PMVLIQKTDT GVSLQTYDDL LAKDCHCI), or an amino acid sequence having a sequence homology of 80% or higher, 85% or higher, 90% or higher, 95% or higher, 96% or higher, 97% or higher, 98% or higher, or 99% or higher with the amino acid sequence in the range that maintains the unique activity and structure of GDF15.

Fc of immunoglobulin

The Fc of the immunoglobulin to be fused with GDF15 or its functional variant plays a role of enhancing the stability of the GDF15 or its functional variant, and for example, it may have an effect of extending the half-life (e.g., in vivo half-life), and/or decreasing renal filtration, etc. In one embodiment, the Fc region of immunoglobulin may be selected from Fc of IgG1 and Fc of IgG4. The Fc region of IgG1 may comprise CH2 domain, CH3 domain, or CH2+CH3 domain of IgG1, and comprise or not comprise a hinge region of IgG1 at the N-terminus. The Fc region of IgG4 may comprise CH2 domain, CH3 domain, or CH2+CH3 domain of IgG4, and comprise or not comprise a hinge region of IgG4 at the N-terminus.

IgG1 may be derived from primates such as humans or rodents such as mice, rats, and the like, and for example, it may be human IgG1 (UniProtKB P01857). IgG4 may be derived from primates such as humans or rodents such as mice, rats, and the like, and for example, it may be human IgG4 (UniProtKB P01861).

The Fc region of IgG1 may comprise CH2 domain, CH3 domain, or CH2+CH3 domain of IgG1, and comprise or not comprise a hinge region of IgG1 at the N-terminus. The Fc region of IgG4 may comprise CH2 domain, CH3 domain, or CH2+CH3 domain of IgG4, and comprise or not comprise a hinge region of IgG4 at the N-terminus.

In one specific embodiment, Fc of IgG1 may be a polypeptide comprising CH2 domain and CH3 domain of human IgG1 (SEQ ID NO: 3), or a polypeptide further comprising a hinge region of human IgG1 (SEQ ID NO: 4) at the N-terminus of the amino acid sequence of the SEQ ID NO: 3. As described herein, the Fc region of IgG1 may be interpreted as comprising an amino acid sequence having a sequence homology of 80% or higher, 85% or higher, 90% or higher, 95% or higher, 96% or higher, 97% or higher, 98% or higher, or 99% or higher with the amino acid sequence of the SEQ ID NO: 3 or 'N-terminus-(SEQ ID NO: 4)-(SEQ ID NO: 3)-C-terminus' in the range that maintains the unique activity and structure of IgG1.

Fc of IgG4 may be a polypeptide comprising CH2 domain and CH3 domain of human IgG4 (SEQ ID NO: 5), or a polypeptide further comprising a hinge region of human IgG4 (SEQ ID NO: 10) at the N-terminus of the amino acid sequence of the SEQ ID NO: 5. As described herein, the Fc region of IgG4 may be interpreted as comprising an amino acid sequence having a sequence homology of 80% or higher, 85% or higher, 90% or higher, 95% or higher, 96% or higher, 97% or higher, 98% or higher, or 99% or higher with the amino acid sequence of the SEQ ID NO: 5 or 'N-terminus-(SEQ ID NO: 10)-(SEQ ID NO: 5)-C-terminus'(or the amino acid sequence of variant Fc of IgG4 described below) in the range that maintains the unique activity and structure of IgG4.

When the Fc region of immunoglobulin is used to extend (in vivo) half-life of the protein linked to its N-terminus or C-terminus, it is important to minimize any effector function of Fc, in order to reduce side-effects by the Fc region of immunoglobulin. In this aspect, the human IgG4 Fc region is advantageous due to low binding ability to Fc$\gamma$R and complement factors, compared to other IgG sub-types. In addition, the human IgG4 Fc region may have reduced effector function by comprising amino acid substitution. The amino acid substitution of the human IgG4 Fc region to reduce the effector function may comprise at least one of substitution of phenylalanine that is the 234$^{th}$ residue of IgG4 (UniprotKB P01861) human to alanine and substitution of the 235$^{th}$ residue leucine to alanine (comprised in the CH2-CH3 domain site of IgG4 Fc; the amino acid residue number is according to EU numbering [Kabat, E.A. et al. (1991) Sequences of Proteins of Immunological Interest, 5$^{th}$ edition, U.S. Dept. of Health and Human Services, Bethesda, MD, NIH Publication no. 91-3242]). In addition, the Fc region of IgG4 may comprise amino acid substitution that stabilizes heavy chain dimer formation and prevents formation of half-IgG4 Fc chain. Such amino acid substitution may comprise substitution of the 228$^{th}$ amino acid residue of the Fc region of human IgG4 (UniprotKB P01861), serine (according to EU numbering; comprised in the hinge region) to proline.

In one specific embodiment, in order to decrease immunogenicity, the Fc region may not comprise mutations other than the mutations described above, but not be limited thereto.

In one specific embodiment, the Fc region of immunoglobulin may comprise CH2-CH3 domain of human IgG4 Fc (SEQ ID NO: 5 (general); SEQ ID NO: 6 (wild type), or SEQ ID NO: 7, 8, or 9 (variant)), or further comprise a hinge region of human IgG4 at the N-terminus of the CH2-CH3 domain (SEQ ID NO: 10 (general); SEQ ID NO: 11 (wild type), or SEQ ID NO: 12 (variant)).

The Fc regions of the immunoglobulins which can be used herein are summarized in the following Table 1:

[Table 1]

| | Region | Amino acid sequence | SEQ ID NO: |
|---|---|---|---|
| Human IgG1-Fc (UniProtK B P01857) | Core Hinge | DKTHT CPPCP | 4 |
| | CH2-CH3 | APELLGGPSV FLFPPKPKDT LMISRTPEVT CVVVDVSHED PEVKFNWYVD GVEVHNAKTK PREEQYNSTY RVVSVLTVLH QDWLNGKEYK CKVSNKALPA PIEKTISKAK GQPREPQVYT LPPSRDELTK NQVSLTCLVK GFYPSDIAVE WESNGQPENN YKTTPPVLDS DGSFFLYSKL TVDKSRWQQG NVFSCSVMHE | 3 |
| | | ALHNHYTQKS LSLSPGX (X is absent or K) | |

(continued)

| | Region | Amino acid sequence | SEQ ID NO: |
|---|---|---|---|
| Human IgG4-Fc (UniProtK B P01861) | Hinge (general) | ESKYGPPCPX CP (X is S or P) | 10 |
| | Hinge (wild type) | ESKYGPPCPS CP (X is S, in SEQ ID NO: 10) | 11 |
| | Hinge (variant) | ESKYGPPCPP CP (X is P, in SEQ ID NO: 10) | 12 |
| | CH2-CH3 (general) | APE**X1X2**GGPSV FLFPPKPKDT LMISRTPEVT CVVVDVSQED PEVQFNWYVD GVEVHNAKTK PREEQFNSTY RVVSVLTVLH QDWLNGKEYK CKVSNKGLPS SIEKTISKAK GQPREPQVYT LPPSQEEMTK NQVSLTCLVK GFYPSDIAVE WESNGQPENN YKTTPPVLDS DGSFFLYSRL TVDKSRWQEG NVFSCSVMHE ALHNHYTQKS LSLSLG**X3** (X1 is F or A, X2 is L or A, X3 is absent or K) | 5 |
| | CH2-CH3 (wild type) | APE**FL**GGPSV FLFPPKPKDT LMISRTPEVT CVVVDVSQED PEVQFNWYVD GVEVHNAKTK PREEQFNSTY RVVSVLTVLH QDWLNGKEYK CKVSNKGLPS SIEKTISKAK GQPREPQVYT LPPSQEEMTK NQVSLTCLVK GFYPSDIAVE WESNGQPENN YKTTPPVLDS DGSFFLYSRL TVDKSRWQEG NVFSCSVMHE ALHNHYTQKS LSLSLGK (X1 is F, X2 is L, X3 is K, in SEQ ID NO: 5) | 6 |
| | CH2-CH3 (variant) | APE**AA**GGPSV FLFPPKPKDT LMISRTPEVT CVVVDVSQED PEVQFNWYVD GVEVHNAKTK PREEQFNSTY RVVSVLTVLH QDWLNGKEYK CKVSNKGLPS SIEKTISKAK GQPREPQVYT LPPSQEEMTK NQVSLTCLVK GFYPSDIAVE WESNGQPENN YKTTPPVLDS DGSFFLYSRL TVDKSRWQEG NVFSCSVMHE ALHNHYTQKS LSLSLG (X1 is A, X2 is A, X3 is absent, in SEQ ID NO: 5) | 7 |
| | CH2-CH3 (variant) | APE**A**LGGPSV FLFPPKPKDT LMISRTPEVT CVVVDVSQED PEVQFNWYVD GVEVHNAKTK PREEQFNSTY RVVSVLTVLH QDWLNGKEYK CKVSNKGLPS SIEKTISKAK GQPREPQVYT LPPSQEEMTK NQVSLTCLVK GFYPSDIAVE WESNGQPENN YKTTPPVLDS DGSFFLYSRL TVDKSRWQEG NVFSCSVMHE ALHNHYTQKS LSLSLG (X1 is A, X2 is L, X3 is absent, in SEQ ID NO: 5) | 8 |

(continued)

| | Region | Amino acid sequence | SEQ ID NO: |
|---|---|---|---|
| | CH2-CH3 (variant) | APEF**A**GGPSV FLFPPKPKDT LMISRTPEVT CVVVDVSQED PEVQFNWYVD GVEVHNAKTK PREEQFNSTY RVVSVLTVLH QDWLNGKEYK CKVSNKGLPS SIEKTISKAK GQPREPQVYT LPPSQEEMTK NQVSLTCLVK GFYPSDIAVE WESNGQPENN YKTTPPVLDS DGSFFLYSRL TVDKSRWQEG NVFSCSVMHE ALHNHYTQKS LSLSLG (X1 is F, X2 is A, X3 is absent, in SEQ ID NO: 5) | 9 |

Fusion polypeptide

The fusion polypeptide provided herein comprises an Fc region of immunoglobulin, and GDF15 or its functional variant linked to the C-terminus of the Fc region of immunoglobulin. The Fc region of immunoglobulin and GDF15 or its functional variant are as described above.

In the fusion polypeptide, the Fc region of immunoglobulin and GDF15 or its functional variant may be linked covalently or non-covalently, or be linked by an appropriate linker (for example, a peptide linker) or directly without a linker. In one embodiment, the peptide linker may be a polypeptide consisting of any amino acids of 1 to 20, 1 to 15, 1 to 10, 2 to 20, 2 to 15, or 2 to 10, and the kinds of the amino acids comprised therein are not limited. In this disclosure, the peptide linker may be a flexible linker, so that the Fc region and GDF15 which are fused to both end of the linker can be independently exhibit its function (e.g., for Fc region, a stabilizing activity, such as, increase in half-life, decrease in renal filtration, and the like). In a specific embodiment, the peptide linker may not be a rigid linker. For example, the peptide linker may comprise at least one amino acid residue selected from the group consisting of Gly, Asn, Ser, Glu, and Lys, and may also comprise neutral amino acids such as Thr and/or Ala, but not limited thereto, and the amino acid sequence suitable to the peptide linker is known in the art.

In one embodiment, the peptide linker may be a GS linker repeatedly comprising one or more of Gly (G) and one or more of Ser (S), and for example, may be (GGGGS)n (n is a repeat count of GGGGS (SEQ ID NO: 13), and is an integer of 1 to 10 or an integer of 1 to 5 (1, 2, 3, 4, or 5)), but not limited thereto. In the GS linker, Gly (Glycine), which is an amino acid having a hydrogen (-H) as a R group, is non-polar and has high degree of freedom (phi, psi angle) and superior mobility; and Ser (Serine), which is an amino acid having -CH2-OH as a R group, has small size and polarity to form a hydrogen bond with water, which is advantageous for maintaining stability, thereby contributing to decrease in non-specific interaction between the GS linker, and GDF15 or Fc region. In addition, the GS linker has flexible structure, thereby being advantageous for reduced immunogenicity. In addition, the GS linker may play a role in spatially separating the Fc region and GDF15, so that their functions are not disturbed by each other. The GS linker may has an amino acid length of 15 to 25 amino acids (n = 3 to 5), 15 to 20 amino acids (n = 3 or 4), 15 to 25 amino acids (n = 4 or 5), or 20 amino acids (n=4). In a specific embodiment, the peptide linker, which is comprised in the fusion polypeptide provided in this description, may not comprise amino acids other than the GS linker [(GGGGS)n (n is an integer from 1 to 10, or an integer from 1 to 5)] as described above.

In an embodiment, the fusion polypeptide may further comprise a peptide linker between the Fc region of immunoglobulin and GDF15 or its functional variant. In one embodiment, the peptide linker may be a GS linker repeatedly comprising one or more of Gly (G) and one or more of Ser (S), and for example, may be (GGGGS)n (n is a repeat count of GGGGS (SEQ ID NO: 13), and is an integer of 1 to 10 or an integer of 1 to 5 (1, 2, 3, 4, or 5)), but not limited thereto.

The fusion polypeptide may be produced recombinantly or be synthesized chemically. When produced recombinantly, the fusion polypeptide may be encoded by one reading frame of which expression is controlled by one transcription initiation regulating factor (for example, promoter, etc.) (represented by "single strand").

In the fusion polypeptide, GDF15 or its functional variant fused with the Fc region of immunoglobulin is characterized by increased in vivo (or in blood) stability (duration) (for example, increased in vivo or in blood half-life) and/or decreased immunogenicity, compared to a case wherein GDF15 or its functional variant is not fused with an Fc region of immunoglobulin, and/or Fc region of immunoglobulin and GDF15 or its functional variant are fused via a linker other than the GS linker as described above. In addition, the GDF15 or its functional variant fused with the Fc region of immunoglobulin is characterized by the improved pharmacological effect (for example, weight loss effect), compared to a case wherein GDF15 or its functional variant is not fused with an Fc region of immunoglobulin.

Fusion polypeptide dimer

A functionally active form of GDF15 is a homodimer form.

Accordingly, other embodiment of the present invention provides a fusion polypeptide dimer in which 2 fusion polypeptides (first fusion polypeptide and second fusion polypeptide) are combined. The first fusion polypeptide comprises an Fc region of the first immunoglobulin and the first GDF15 or its functional variant, and the second fusion polypeptide comprises an Fc region of the second immunoglobulin and the second GDF15 or its functional variant. In the dimer, in the first fusion polypeptide and the second fusion polypeptide, the first GDF15 or its functional variant and the second GDF15 or its functional variant may be linked by a covalent bond (for example, disulfide bond, etc.). In addition, in the dimer, optionally, the Fc region of the first immunoglobulin and the Fc region of the second immunoglobulin may be linked by a covalent bond (for example, disulfide bond, etc.) or a non-covalent bond (for example, knob & hole, electrostatic interaction, hydrophobic interaction, etc.) (See FIG. 1). The Fc region of immunoglobulin and GDF15 or its functional variant are as described above, and the Fc region of the first immunoglobulin and the Fc region of the second immunoglobulin, and the first GDF15 or its functional variant and the second GDF15 or its functional variant may be same or different each other.

In one embodiment, the Fc region of the first immunoglobulin and the Fc region of the second immunoglobulin, and the first GDF15 or its functional variant and the second GDF15 or its functional variant, and the first fusion polypeptide and the second fusion polypeptide formed by linking thereof, comprised in the fusion polypeptide dimer, is a single strand (chain; for example, a polypeptide encoded by one reading frame) form, respectively, and the structure in which at least one of the Fc region of the first immunoglobulin and the Fc region of the second immunoglobulin, and the first GDF15 or its functional variant and the second GDF15 or its functional variant, and the first fusion polypeptide and the second fusion polypeptide formed by linking thereof is a dimer form (for example, the Fc region of the first or second immunoglobulin comprised in the first or second fusion polypeptide is linked by a disulfide bond of 2 strands (2 molecules), and the like), is excluded.

The N-terminus of the monomer in the dimer structure of GDF15 is exposed to be fused with other protein, and the C-terminus is positioned where it cannot be fused with other protein. Therefore, to form a fusion polypeptide while maintaining the GDF15 dimer form, a fusion partner (namely, Fc region of immunoglobulin) is preferable to be linked to the N-terminus of GDF15.

GDF15 forms a GDF15-GFRAL complex structure by combining with its receptor GFRAL (GDNF family receptor alpha-like; for example, GenBank Accession no. NP_997293.2). In the complex structure, the GDF15 monomer and GFRAL monomer are combined respectively, and the N-terminus of GDF15 is positioned in the middle part of the GDF15 dimer. 4 amino acid residues at the N-terminus which can be fused to GDF15 are not observed in the X-ray structure, and therefore it is predicted that it does not have a specific structure. 14 amino acid residues at the N-terminus of GDF15 is fixed by Cys7-Cys14 disulfide bond, and it is not involved in interaction between monomers in the GDF15 dimer. In addition, 14 amino acid residues at the N-terminus of GDF15 are structurally positioned at a distance that cannot interact (bind) to the GFRAL receptor, and thus it is expected that there is no effect on binding of GDF15 to the GFRAL receptor, although 14 amino acid residues at the N-terminus of GDF15 are deleted.

The GDF15 monomer has 4 disulfide bonds in the molecule and one disulfide bone between dimer molecules. The disulfide bonds in the molecule is assumed to stabilizes the structure, and the disulfide bond combining Cys7-Cys14 is positioned at the edge of the monomer structure and acts to fix the loop structure of the N-terminus. Since 14 amino acid residues at the N-terminus do not play a direct role in GFRAL receptor binding, if one or more of them are removed, it will not affect the function and structure of GDF15, but Cys15 forms a disulfide bond with Cys88, and therefore, if Cys15 is removed, it may affect the three-dimensional structure of GDF15. Therefore, the removable region while maintaining the function and structure of GDF15 may be 14 amino acids at the N-terminus (namely, in SEQ ID NO: 1, at least one of 14 amino acids from Ala1 to Cys14, for example, at least one from the N-terminus in order (1, 2, 3, 4, 5, 6, 7, 8, 9, 10, 11, 12, 13, or 14)). In one embodiment, the GDF15 monomer may be wild type (SEQ ID NO: 1) or a form in which 14 amino acids at the N-terminus are deleted in the wild type (AN14GDF15; SEQ ID NO: 2).

In the complex structure in which wtGDF15 (SEQ ID NO: 1) or ΔN14GDF15 (the form in which 14 amino acids at the N-terminus are deleted in SEQ ID NO: 1) is combined with its receptor GFRAL, wt(wild type)GDF15 N-terminus (1-14 amino acid residues) part is fixed by Cys7-Cys14 disulfide bond, and the N-terminus is exposed in the direction of bottom left and bottom right, respectively. When 14 amino acids at the N-terminus are removed in wtGDF15, the N-terminus is exposed in the direction of top left and top right, respectively.

Due to such a structure, when a fusion protein is formed by fusing an Fc protein of an immunoglobulin with GDF15, ΔN14GDF15 in which 14 amino acid residues at the N-terminus are removed and the C-terminus of the Fc protein can be morphologically naturally linked (See FIG. 3a). On the other hand, in case of wtGDF15, the N-terminus may be positioned in the direction little difficult to combine with the Fc protein (See FIG. 3b).

In the complex structure in which Fc-ΔN14GDF15 or Fc-wtGDF15 is combined to GFRAL, GDF15 and Fc are a functionally dimer form, and Fc-ΔN14GDF15 has a structural arrangement easy to form an Fc dimer and a ΔN14GDF15 dimer, respectively. Therefore, Fc-ΔN14GDF15 may be advantageous than Fc-wtGDF15 in aspect of dimer formation without being interrupted in formation of a complex with GFRAL

The in vivo (in blood) half-life in mammals of the GDF15 or its functional variant comprised in the fusion polypeptide or fusion polypeptide dimer provided herein may be increased about 1.5 time or more, about 2 times or more, about 2.5 times or more, about 3 times or more, about 3.5 times or more, about 4 times or more, about 4.5 times or more, about 5 times or more, about 5.5 times or more, about 6 times or more, about 7 times or more, about 8 times or more, about 9 times or more, or about 10 times, compared to GDF15 or its functional variant not fused with an Fc region of immunoglobulin.

As described above, GDF15 or its functional variant in a fusion polypeptide form which is fused with an Fc region of immunoglobulin has an advantage of long administration intervals, compared to GDF15 or its functional variant not fused with an Fc region of immunoglobulin, by the increased half-life of GDF15 or its functional variant.

<u>Production of fusion polypeptide</u>

The fusion polypeptide comprising GDF15 or its functional variant and an Fc region of immunoglobulin may be produced by common chemical synthesis methods or recombinant methods, and may be not naturally occurring.

Herein, the term "vector" means an expression means to express a target gene in a host cell, and for example, may be selected from the group consisting of plasmid vector, cosmid vector and virus vector such as bacteriophage vector, adenovirus vector, retrovirus vector and adeno-associated virus vector, and the like. In one embodiment, the vector usable in the recombinant vector may be prepared on the basis of plasmid (for example, pcDNA series, pSC101, pGV1106, pACYC177, ColE1, pKT230, pME290, pBR322, pUC8/9, pUC6, pBD9, pHC79, pIJ61, pLAFR1, pHV14, pGEX series, pET series, pUC19, etc.), phage (for example, λgt4λB, λ-Charon, λΔz1, M13, etc.) or virus (for example, SV40, etc.), but not limited thereto.

In the recombinant vector, a nucleic acid molecule encoding the fusion polypeptide may be operatively linked to a promoter. The term "operatively linked" means a functional binding of a nucleic acid expression regulating sequence (for example, promoter sequence) and other nucleic acid sequence. The regulating sequence may regulate transcription and/or translation of other nucleic acid sequence by being "operatively linked".

The recombinant vector may be typically constructed as a vector for cloning or an expression vector for expression. As the expression vector, common one used for expressing foreign protein in plants, animals, or microorganisms, in the art may be used. The recombinant vector may be constructed by various method known in the art.

The recombinant vector may be expressed using a eukaryote as a host. When expressing using an eukaryote as a host, the recombinant vector may comprise a nucleic acid molecule to be expressed and an aforementioned promoter, ribosome binding site, secretion signal sequence (See Korean Patent Publication No. 2015-0125402) and/or in addition to transcription/translation terminator sequence, a replication origin operating in eukaryotes such as f1 replication origin, SV40 replication origin, pMB1 replication origin, adeno replication origin, AAV replication origin and/or BBV replication origin, and the like, but not limited thereto. In addition, a promoter derived from genome of a mammal cell (for example, metallothionein promoter) or promoter derived from a mammal virus (for example, adenovirus late promoter, vaccinia virus 7.5K promoter, SV40 promoter, cytomegalovirus promoter, fusion promoter (KR 10-1038126 or KR 10-1868139), *tk* promoter of HSV) may be used, and all secretion signal sequences commonly usable as a secretion signal sequence may be used, and for example, the secretion signal sequence disclosed in Korean Patent Publication No. 2015-0125402 may be used, but not limited thereto, and it may comprise a polyadenylated sequence as the transcription terminator sequence.

The recombinant cell may be obtained by introducing (transforming or transfecting) the recombinant vector to an appropriate host cell. The host cell may be selected from all eukaryotes which can stably and continuously clone or express the recombinant vector. The eukaryote usable as a host includes yeasts *(Saccharomyces cerevisiae),* insect cells, plant cells, and animal cells, and the like, and for example, includes mice (for example, COP, L, C127, Sp2/0, NS-0, NS-1, At20, or NIH3T3), rats (for example, PC12, PC12h, GH3, or MtT), hamsters (for example, BHK, CHO, GS gene defect CHO, or DHFR gene defect CHO), monkeys (for example, COS (COS1, COS3, COS7, etc.), CV1 or Vero), humans (for example, HeLa, HEK-293, retina-derived PER-C6, cells derived from diploid fibroblasts, myeloma cells or HepG2), other animal cells (for example, MDCK, etc.), insect cells (for example, Sf9 cell, Sf21 cell, Tn-368 cell, BTI-TN-5B1-4 cell, etc.), hybridoma, and the like, but not limited thereto.

By expressing the nucleic acid molecule encoding the fusion polypeptide provided herein in an aforementioned appropriate host cell, compared to the not fused form, GDF15 or its functional variant, or a fusion polypeptide or fusion polypeptide dimer comprising thereof with enhanced in vivo stability may be prepared. The method for preparation of the fusion polypeptide or fusion polypeptide dimer may comprise culturing a recombinant cell comprising the nucleic acid molecule. The culturing may be performed under a common culturing condition. In addition, the method for preparation may further comprise isolating and/or purifying a fusion polypeptide or fusion polypeptide dimer from culture, after the culturing.

The delivery (introduction) of the nucleic acid molecule or a recombinant vector comprising the same into a host cell may use delivery methods widely known in the art. The delivery method may use for example, microinjection, calcium phosphate precipitation, electroporation, liposome-mediated transfection and gene bombardment, and the like, when the host cell is a eukaryote, but not limited thereto.

The method for selecting the transformed (recombinant vector introduced) host cell may be easily conducted according to the methods widely known in the art, using a phenotype expressed by a selection marker. For example, when the selection marker is a specific antibioticresistant gene, a recombinant cell in which a recombinant vector is introduced may be easily selected by culturing in a medium containing the antibiotic.

## Medical Uses

A composition (pharmaceutical composition or health functional food composition) for weight loss, dietary control (reducing the size of meals) or prevention, improvement, alleviation and/or treatment of metabolic disease, comprising at least one selected from the group consisting of a fusion polypeptide, a fusion polypeptide dimer, a fusion polypeptide-encoding nucleic acid molecule, a recombinant vector comprising the nucleic acid molecule and a recombinant cell comprising the recombinant vector; and/or

a weight loss method, a method of dietary control (reducing the size of meals), or a method for prevention, improvement, alleviation and/or treatment of metabolic disease, comprising administering a pharmaceutically effective dose of at least one selected from the group consisting of a fusion polypeptide, a fusion polypeptide dimer, a fusion polypeptide-encoding nucleic acid molecule, a recombinant vector comprising the nucleic acid molecule and a recombinant cell comprising the recombinant vector, to a subject in need of prevention, improvement, alleviation and/or treatment of metabolic disease are provided.

The metabolic disease means all diseases caused by metabolic disorders, and may be selected from the group consisting of obesity, diabetes (for example, type II diabetes), nonalcoholic fatty liver disease (for example, nonalcoholic steatohepatitis (NASH), etc.) and the like.

As used herein, the pharmaceutically effective dose means a content or dose of an active ingredient capable of obtaining a desired effect. The content or dose of the active ingredient (at least one selected from the group consisting of a fusion polypeptide comprising GDF15 or its functional variant and an Fc region of immunoglobulin, a fusion polypeptide dimer, a fusion polypeptide-encoding nucleic acid molecule, a recombinant vector comprising the nucleic acid molecule, and a recombinant cell comprising the recombinant vector) in the pharmaceutical composition may be variously prescribed by factors such as formulation method, administration method, patients' age, weight, gender, morbidity, food, administration time, administration interval, administration route, excretion rate and reaction sensitivity. For example, the single dose of the active ingredient may be in a range of 0.001 to 1000 mg/kg, 0.01 to 100 mg/kg, 0.01 to 50 mg/kg, 0.01 to 20 mg/kg, 0.01 to 10mg/kg, 0.01 to 5mg/kg, 0.1 to 100 mg/kg, 0.1 to 50 mg/kg, 0.1 to 20 mg/kg, 0.1 to 10mg/kg, 0.1 to 5mg/kg, 1 to 100 mg/kg, 1 to 50 mg/kg, 1 to 20 mg/kg, 1 to 10mg/kg, or 1 to 5mg/kg, but not limited thereto. In other embodiment, the content of the active ingredient in the pharmaceutical composition may be 0.01% by weight to 99.9% by weight, 0.01% by weight to 90% by weight, 0.01% by weight to 80% by weight, 0.01% by weight to 70% by weight, 0.01% by weight to 60% by weight, 0.01% by weight to 50% by weight, 0.01% by weight to 40% by weight, 0.01% by weight to 30% by weight, 1% by weight to 99.9% by weight, 1% by weight to 90% by weight, 1% by weight to 80% by weight, 1% by weight to 70% by weight, 1% by weight to 60% by weight, 1% by weight to 50% by weight, 1% by weight to 40% by weight, 1% by weight to 30% by weight, 5% by weight to 99.9% by weight, 5% by weight to 90% by weight, 5% by weight to 80% by weight, 5% by weight to 70% by weight, 5% by weight to 60% by weight, 5% by weight to 50% by weight, 5% by weight to 40% by weight, 5% by weight to 30% by weight, 10% by weight to 99.9% by weight, 10% by weight to 90% by weight, 10% by weight to 80% by weight, 10% by weight to 70% by weight, 10% by weight to 60% by weight, 10% by weight to 50% by weight, 10% by weight to 40% by weight, or 10% by weight to 30% by weight, based on the total pharmaceutical composition weight.

The administration interval (time interval between 2 adjacent doses) of the active ingredient or pharmaceutical composition comprising the same provided herein may be adjusted by the concentration or status (variation, etc.) of the active ingredient or patients' condition or symptoms, and the like, and for example, it may be 2 days or more, 3 days or more, 4 days or more, 5 days or more, 6 days or more, 7 days or more, 8 days or more, 9 days or more, 10 days or more, 2 weeks or more, 3 weeks or more, 4 weeks or more, 6 weeks or more, 8 weeks or more, 10 weeks or more, or 12 weeks or more. The maximum administration interval may be about 2 weeks, about 3 weeks, about 4 weeks, about 1 month, about 2 months, or about 3 months, but not limited thereto, and it may be increased or reduced by the concentration or status (variation, etc.) of the active ingredient or patients' condition or symptoms, and the like. In one specific embodiment, the administration interval may be appropriately prescribed within about 1 week to about 3 months.

In addition, the pharmaceutical composition may further comprise a pharmaceutically acceptable carrier, in addition to the active ingredient. The carrier is commonly used for formulation of drugs comprising protein, nucleic acids or cells, and it may be at least one selected from the group consisting of lactose, dextrose, sucrose, sorbitol, mannitol, starch, acacia gum, calcium phosphate, alginate, gelatin, calcium silicate, microcrystalline cellulose, polyvinyl pyrrolidone, cellulose, water, syrup, methyl cellulose, methyl hydroxy benzoate, propyl hydroxy benzoate, talc, magnesium stearate, mineral oil, and the like, but not limited thereto. The pharmaceutical composition may further comprise at least one selected from the group consisting of a diluent, an excipient, a lubricant, a wetting agent, a sweetener, a flavoring agent, an emulsifier, a suspending agent, a preservative, and the like, commonly used for preparation of a pharmaceutical composition.

The administration subject of the pharmaceutical composition may be mammals including primates such as humans, monkeys, etc., rodents such as mice, rats, etc., and the like, or cells, tissue, cell culture or tissue culture derived therefrom.

The pharmaceutical composition may be administered by oral administration or parenteral administration, or be administered by contacting to a cell, tissue or body fluid. Specifically, in case of parenteral administration, it may be administered by subcutaneous injection, intramuscular injection, intravenous injection, intraperitoneal injection, endothelial administration, topical administration, intranasal administration, intrapulmonary administration and rectal administration, and the like. In case of oral administration, the oral composition should be formulated to coat an active agent or to protect it from degradation in stomach, as proteins or peptides are digested.

In addition, the pharmaceutical composition may be formulated in a form of solution, suspension, syrup or emulsion in oil or an aqueous medium, or in a form of extract, powder, granules, tablets or capsules, or the like, and may further comprise a dispersing agent or stabilizing agent for formulation.

Another embodiment provides a method of increasing in vivo duration of GDF15 or its functional variant comprising fusing (or linking or combining) GDF15 or its functional variant with an Fc region of immunoglobulin. In one specific embodiment, the fusing may comprise fusing (or linking or combining) the Fc region of immunoglobulin to the N-terminus of GDF15 or its functional variant with or without a linker. Another embodiment provides a method of decreasing immunogenicity of GDF15, an Fc region of immunoglobulin, or a fusion polypeptide comprising both of them comprising fusing (or linking or combining) GDF15 or its functional variant with an Fc region of immunoglobulin with a flexible linker. The step of fusing (or linking or combining) may be conducted in vitro.

[ADVANTAGEOUS EFFECTS]

GDF15 fused with an Fc region of immunoglobulin or its functional variant provided in the present invention has a longer duration when administered in a body, compared to the case where it is not fused with an Fc region of immunoglobulin, and thus the administration interval can be increased and thereby the dose can be reduced, and therefore it has an advantageous effect in terms of convenience of administration and/or economical aspect, and it also has an excellent pharmacological effect, and thus it can be usefully applied to fields requiring treatment of GDF15 or its functional variant.

[BRIEF DESCRIPTION OF THE DRAWINGS]

FIG. 1 is a mimetic diagram of the fusion polypeptide according to one example.

FIG. 2 shows the amino acid sequence of GDF15 (wild type; mature form; SEQ ID NO: 1) (from N-terminus to C-terminus).

FIG. 3a and FIG. 3b show the structure of the complex in which Fc-ΔN14GDF15 (3a) or Fc-wtGDF15 (3b) binds to GFRAL

FIG. 4 is a mimetic diagram schematically showing the fusion polypeptide gene according to the example.

FIG. 5 is a graph showing the weight change of the IgG1-GDF (CRL) administration group and HIgG1-GDF (CRL) administration group.

FIG. 6 is a graph showing the weight change rate at 7 days after administration of the IgG1-GDF (CRL) administration group and HIgG1-GDF (CRL) administration group.

FIG. 7 is a graph showing the weight change of the IgG4-GDF15 administration group and IgG4-GDF(CRL) administration group.

FIG. 8 is a graph showing the weight change rate at 7 days (blue bar) and 14 days (red bar) after administration of the IgG4-GDF15 administration group and IgG4-GDF(CRL) administration group.

FIG. 9 is a graph showing the cumulative feed intake by 7 days after administration of the IgG1-GDF (CRL) administration group and HIgG1-GDF (CRL) administration group.

FIG. 10 is a graph showing the cumulative feed intake by 7 days, 14 days and 19 days after administration of the IgG4-GDF15 administration group and IgG4-GDF(CRL) administration group.

FIG. 11 is a graph showing the change in the fusion polypeptide concentration in blood (in serum) according to the elapsed time after administration of the fusion polypeptide.

[DETAILED DESCRIPTION OF THE EMBODIMENTS]

Hereinafter, the present invention will be described in more detail by the following examples. However, these examples are intended to illustrate the present invention only, but the scope of the present invention is not limited by these examples.

## Example 1: Preparation of fusion polypeptide

### 1.1. Cloning and culturing of gene encoding fusion polypeptide

IgG1-GDF(CRL), HIgG1-GDF(CRL), IgG4-GDF, HIgG4-GDF, IgG4-GDF(CRL), and HIgG4-GDF(CRL) (See FIG. 1) which were fusion polypeptides fused with Mature GDF15 (represented by GDF or GDF15; SEQ ID NO: 1), or GDF15 variant (represented by GDF(CRL); 14 amino acids at the N-terminus were deleted: SEQ ID NO: 2) that had a target polypeptide of immunoglobulin Fc (IgG1-Fc (not comprising hinge: SEQ ID NO: 3); HIgG1-Fc (comprising hinge; [SEQ ID NO: 4]-[SEQ ID NO: 3]), Mutated IgG4-Fc (not comprising hinge; SEQ ID NO: 7) or Mutated HIgG4-Fc (comprising hinge: [SEQ ID NO: 12]-[SEQ ID NO: 7])) comprising or not comprising hinge, were prepared. The amino acid sequences of each part comprised in the fusion polypeptide were summarized in Table 2, Table 3 and Table 4 below.

[Table 2]

| IgG1-GDF(CRL) (not comprising hinge) & HIgG1-GDF(CRL) (comprising hinge) (direction from N-terminus to C-terminus) | | | |
|---|---|---|---|
| | | Amino acid sequence | SEQ ID NO: |
| Signal Peptide | | MHRPEAMLLL LTLALLGGPT WA | 17 |
| | Hinge | DKTHTCPPCP | 4 |
| IgG1 Fc | CH2-CH3 | APELLGGPSV FLFPPKPKDT LMISRTPEVT CVVVDVSHED PEVKFNWYVD GVEVHNAKTK PREEQYNSTY RVVSVLTVLH QDWLNGKEYK CKVSNKALPA PIEKTISKAK GQPREPQVYT LPPSRDELTK NQVSLTCLVK GFYPSDIAVE WESNGQPENN YKTTPPVLDS DGSFFLYSKL TVDKSRWQQG NVFSCSVMHE ALHNHYTQKS LSLSPG | 3 |
| Linker | | GGGGSGGGGS GGGGSGGGGS | 18 |
| GDF( CRL) | | CRLHTVRASL EDLGWADWVL SPREVQVTMC IGACPSQFRA ANMHAQIKTS LHRLKPDTVP APCCVPASYN PMVLIQKTDT GVSLQTYDDL LAKDCHCI | 2 |

[Table 3]

| IgG4-GDF15 (not comprising hinge) & HIgG4-GDF15 (comprising hinge) (direction from N-terminus to C-terminus) | | | |
|---|---|---|---|
| | | Amino acid sequence | SEQ ID NO: |
| Signal Peptide | | MHRPEAMLLL LTLALLGGPT WA | 17 |
| Mutated IgG4 Fc | Hinge | ESKYGPPCPP CP | 12 |
| | CH2-CH3 | APEAAGGPSV FLFPPKPKDT LMISRTPEVT CVVVDVSQED PEVQFNWYVD GVEVHNAKTK PREEQFNSTY RVVSVLTVLH QDWLNGKEYK CKVSNKGLPS SIEKTISKAK GQPREPQVYT LPPSQEEMTK NQVSLTCLVK GFYPSDIAVE WESNGQPENN YKTTPPVLDS DGSFFLYSRL TVDKSRWQEG NVFSCSVMHE ALHNHYTQKS LSLSLG | 7 |
| Linker | | GGGGSGGGGS GGGGSGGGGS | 18 |
| GDF15 | | ARNGDHCPLG PGRCCRLHTV RASLEDLGWA DWVLSPREVQ VTMCIGACPS QFRAANMHAQ IKTSLHRLKP DTVPAPCCVP ASYNPMVLIQ KTDTGVSLQT YDDLLAKDCH CI | 1 |

[Table 4]

| IgG4-GDF(CRL) (not comprising hinge) & HIgG4-GDF(CRL) (comprising hinge) (direction from N-terminus to C-terminus) | | | |
|---|---|---|---|
| | | Amino acid sequence | SEQ ID NO: |
| Signal Peptide | | MHRPEAMLLL LTLALLGGPT WA | 17 |
| Mutated IgG4 Fc | Hinge | ESKYGPPCPP CP | 12 |
| | CH2-CH3 | APEAAGGPSV FLFPPKPKDT LMISRTPEVT CVVVDVSQED PEVQFNWYVD GVEVHNAKTK PREEQFNSTY RVVSVLTVLH QDWLNGKEYK CKVSNKGLPS SIEKTISKAK GQPREPQVYT LPPSQEEMTK NQVSLTCLVK GFYPSDIAVE WESNGQPENN YKTTPPVLDS DGSFFLYSRL TVDKSRWQEG NVFSCSVMHE ALHNHYTQKS LSLSLG | 7 |
| Linker | | GGGGSGGGGS GGGGSGGGGS | 18 |
| GDF15(CRL) | | CRLHTVRASL EDLGWADWVL SPREVQVTMC IGACPSQFRA ANMHAQIKTS LHRLKPDTVP APCCVPASYN PMVLIQKTDT GVSLQTYDDL LAKDCHCI | 2 |

### 1.1.1 Preparation of recombinant expression vector

#### 1.1.1.1. Mature GDF15

To obtain a gene encoding Mature GDF15, referring to the amino acid of UniprotKB Q99968, a gene encoding Mature GDF15 (SEQ ID NO: 14) was synthesized in Bioneer.

SEQ ID NO: 14 (339bp)

1 GCCCGGAACG GCGACCACTG CCCCCTGGGG CCCGGACGGT GCTGCCGGCT

51 GCACACCGTG CGGGCCTCCC TGGAGGACCT GGGCTGGGCC GACTGGGTGC

101 TGTCCCCAAG GGAGGTGCAA GTGACCATGT GCATCGGCGC CTGCCCATCT

151 CAGTTCCGGG CCGCCAACAT GCACGCTCAG ATCAAGACCA GCCTGCACCG

201 GCTGAAGCCC GACACCGTGC CCGCCCCCTG CTGCGTGCCC GCCTCCTACA

251 ACCCCATGGT GCTGATTCAG AAGACCGACA CCGGCGTGAG CCTGCAGACC

301 TACGACGACC TGCTGGCCAA GGACTGCCAC TGCATCTAA

(The underlined part is GDF (CRL))

#### 1.1.1.2. IgG1-Fc

A gene coding human IgG1 Fc comprising hinge or a gene coding human IgG1 Fc not comprising hinge was obtained using by a plasmid comprising a gene coding core hinge of human IgG1 and IgG1 Fc by PCR.

SEQ ID NO: 15 (678bp)

1 **GACAAAACTC ACACATGCCC ACCGTGCCCA** GCACCTGAAC TCCTGGGGGG

51 ACCGTCAGTC TTCCTCTTCC CCCCAAAACC CAAGGACACC CTCATGATCT

101 CCCGGACCCC TGAGGTCACA TGCGTGGTGG TGGACGTGAG CCACGAAGAC

151 CCTGAGGTCA AGTTCAACTG GTACGTGGAC GGCGTGGAGG TGCATAATGC

201 CAAGACAAAG CCGCGGGAGG AGCAGTACAA CAGCACGTAC CGTGTGGTCA

251 GCGTCCTCAC CGTCCTGCAC CAGGACTGGC TGAATGGCAA GGAGTACAAG

301 TGCAAGGTCT CCAACAAAGC CCTCCCAGCC CCCATCGAGA AAACCATCTC

351 CAAAGCCAAA GGGCAGCCCC GAGAACCACA GGTGTATACC CTGCCCCCAT

401 CCCGGGATGA GCTGACCAAG AACCAGGTCA GCCTGACCTG CCTGGTCAAA

451 GGCTTCTATC CCAGCGACAT CGCCGTGGAG TGGGAGAGCA ATGGGCAGCC

501 GGAGAACAAC TACAAGACCA CGCCTCCCGT GCTGGACTCC GACGGCTCCT

551 TCTTCCTCTA CAGCAAGCTC ACCGTGGACA AGAGCAGGTG GCAGCAGGGG

601 AACGTCTTCT CATGCTCCGT GATGCATGAG GCTCTGCACA ACCACTACAC

651 GCAGAAGAGC CTCTCCCTGT CTCCGGGT

(The underlined part is a gene coding IgG1 Core Hinge)

**1.1.1.3. IgG4-Fc**

A gene coding human IgG4 Fc comprising hinge or a gene coding human IgG4 Fc not comprising hinge was obtained using by a plasmid comprising a gene coding hinge of human IgG4 and IgG4 Fc by PCR.

SEQ ID NO: 16 (684bp)

1 **GAGTCCAAAT ATGGTCCCCC ATGCCCACCC TGCCCA**GCAC CTGAGGCCGC

51 CGGGGGACCG TCAGTCTTCC TCTTCCCCCC AAAACCCAAG GACACCCTCA

101 TGATCTCCCG GACCCCTGAG GTCACGTGCG TGGTGGTGGA CGTGTCCCAG

151 GAGGACCCCG AGGTGCAGTT CAACTGGTAC GTGGACGGCG TGGAGGTGCA

201 CAACGCCAAG ACCAAGCCCC GGGAGGAGCA GTTCAACTCC

ACCTACCGGG

251 TGGTGTCCGT GCTGACCGTG CTGCACCAGG ACTGGCTGAA CGGCAAGGAG

301 TACAAGTGCA AGGTGTCCAA CAAGGGCCTG CCCTCCTCCA TCGAGAAGAC

351 CATCTCCAAG GCCAAGGGCC AGCCCCGGGA GCCCCAGGTG TACACCCTGC

401 CCCCCTCCCA GGAGGAGATG ACCAAGAACC AGGTGTCCCT GACCTGCCTG

451 GTGAAGGGCT TCTACCCCTC CGACATCGCC GTGGAGTGGG AGTCCAACGG

501 CCAGCCCGAG AACAACTACA AGACCACCCC CCCCGTGCTG GACTCCGACG

551 GCTCCTTCTT CCTGTACTCC CGGCTGACCG TGGACAAGTC CCGGTGGCAG

601 GAGGGCAACG TGTTCTCCTG CTCCGTGATG CACGAGGCCC TGCACAACCA

651 CTACACCCAG AAGTCCCTGT CCCTGTCCCT GGGC

(The underlined part is a gene coding IgG4 Hinge)

**1.1.1.4. Preparation of expression vector**

pDHDD-D1G1 (comprising the promoter of KR10-1868139B1) which was a variant of pcDNA3.1(+) (Invitrogen, Cat. No. V790-20) was cut by BamHI and NotI, and the genes (mature GDF15, IgG1-Fc, IgG4-Fc) were combined thereto to insert the gene having the following structure (See FIG. 4), thereby preparing each recombinant vector.

pHIgGI-GDF(CRL)

'(N-terminus)-[BamHI restriction site-signal peptide (SEQ ID NO: 17)-IgG1 Core Hinge (SEQ ID NO: 4)-IgG1 CH2-CH3 (SEQ ID NO: 3)-GS Linker (SEQ ID NO: 18)-GDF (CRL) (SEQ ID NO: 2)- NotI restriction site]-(C-terminus)'

pIgG1-GDF(CRL)

'(N-terminus)-[BamHI restriction site-signal peptide (SEQ ID NO: 17)-IgG1 CH2-CH3 (SEQ ID NO: 3)-GS Linker (SEQ ID NO: 18)-GDF (CRL) (SEQ ID NO: 2)- NotI restriction site]-(C-terminus)'

pHIgG4-GDF15

'(N-terminus)-[BamHI restriction site-signal peptide (SEQ ID NO: 17)-IgG4 Hinge (SEQ ID NO: 12)-IgG4 CH2-CH3 (SEQ ID NO: 7)-GS Linker (SEQ ID NO: 18)-GDF15 (SEQ ID NO: 1)-NotI restriction site]-(C-terminus)'

pIgG4-GDF15

'(N-terminus)-[BamHI restriction site-signal peptide (SEQ ID NO: 17)-IgG4 CH2-CH3 (SEQ ID NO: 7)-GS Linker (SEQ ID NO: 18)-GDF15 (SEQ ID NO: 1)- NotI restriction site]-(C-terminus)'

pHIgG4-GDF(CRL)

'(N-terminus)-[BamHI restriction site-signal peptide (SEQ ID NO: 17)-IgG4 Hinge (SEQ ID NO: 12)-IgG4 CH2-CH3 (SEQ ID NO: 7)-GS Linker (SEQ ID NO: 18)-GDF(CRL) (SEQ ID NO: 2)- NotI restriction site]-(C-terminus)'

pIgG4-GDF(CRL)

'(N-terminus)-[BamHI restriction site-signal peptide (SEQ ID NO: 17)- IgG4 CH2-CH3 (SEQ ID NO: 7)-GS Linker (SEQ ID NO: 18)-GDF(CRL) (SEQ ID NO: 2)- NotI restriction site] -(C-terminus)'

**1.1.2. Culturing of gene encoding fusion polypeptide**

The prepared recombinant expression vectors, pHIgG1-GDF(CRL), pIgG1-GDF(CRL), pHIgG4-GDF(CRL), pIgG4-GDF(CRL), pHIgG4-GDF and pIgG4-GDF were introduced to ExpiCHO-S™ cell (Thermo Fisher Scientific), and were cultured in ExpiCHO Expression Medium (Thermo Fisher Scientific; 400 mL) for 12 days (Fed-Batch Culture; Day 1 & Day 5 Feeding), to express the fusion polypeptides, HIgG1-GDF(CRL), IgG1-GDF(CRL), HIgG4-GDF(CRL), IgG4-GDF(CRL), HIgG4-GDF and IgG4-GDF.

**1.2. Purification of fusion polypeptide**

The fusion polypeptide was purified from the cell culture prepared in Example 1.1 using Protein A Affinity Chromatography.

At first, the culture solution of the cell-removed fusion polypeptide was filter with a 0.22 μm filter. A column in which MabSelect SuRe™ pcc (GE Healthcare Life Sciences) resin was packed was equipped to AKTA™ Pure (GE Healthcare Life Sciences), and Phosphate Buffered Saline (PBS, 10 mM Sodium Phosphate, 150 mM NaCl, pH 7.4) was flowed to equilibrate the column. After injecting the culture solution filtered with the 0.22 μm filter to the equilibrated column, PBS was flowed again to wash the column. After finishing washing of the column, eluting buffer (0.1 M Sodium Citrate pH 3.5) was flowed to the column to elute the target fusion polypeptide. 1M Tris pH 8.5 was added immediately to the eluted solution so as to be neutral pH. Among eluted fractions, fractions with high concentration of the fusion polypeptide and high purity were collected, and were stored frozen.

For an animal experiment, using Amicon Ultra Filter Device (MWCO 10K, Merck) and a centrifuge, the eluted fraction sample comprising the fusion polypeptide was concentrated with PBS or 20 mM Tris pH 8.0, 150 mM NaCl and buffer exchange was conducted.

The quantitative analysis of the fusion polypeptide was performed by measuring the absorbance at 280 nm and 340 nm in UV Spectrophotometer (G113A, Agilent Technologies), and calculating the protein concentration by the following equation. As the extinction coefficient of each material, the value theoretically calculated using the amino acid sequence (Table 5) was used.

$$Protein\ concentration\ (mg/mL) = \frac{Absorbance\ (A_{280nm} - A_{340nm})}{*\ Extinction\ Coefficient} \times Dilution\ Factor$$

**[0086]** * Extinction coefficient (0.1%): a theoretical absorbance at 280 nm, assuming that the protein concentration is 0.1% (1g/L), and all cysteines on the primary sequence are oxidized to form a disulfide bond, which is calculated by ProtParam tool (https://web.expasy.org/protparam/).

[Table 5]

| Extinction coefficient of fusion polypeptide | |
|---|---|
| Sample name | Extinction coefficient (0.1%, 1 mg/mL) |
| IG1-GDF (CRL) | 1.317 |

(continued)

| Extinction coefficient of fusion polypeptide | |
|---|---|
| Sample name | Extinction coefficient (0.1%, 1 mg/mL) |
| HIgG1-GDF (CRL) | 1.22 |
| IG4-GDF (CRL) | 1.334 |
| IgG4-GDF | 1.287 |

**Example 2. Pharmacological effect of fusion polypeptide (in vivo)**

**2.1. Test process**

The pharmacological effect of the fusion polypeptide produced and purified in Example 1 was tested in mice (C57BL/6J, 6 weeks, male, 100 mice; RaonBio).

In this example, DI0 mouse model (Mouse, C57BL/6J -DIO, male, 100 mice, 14 weeks (8-week feeding of obesity feed) in which the high-fat diet was fed to the C57BL/6J mice for 8 weeks to induce obesity was used. The DI0 mouse model exhibits clinical characteristics of type 2 diabetes such as hyperlipidemia, insulin resistance, hyperglycemia, and the like, and therefore it is an animal model widely used for evaluation of improvement of diabetes and insulin resistance, and baseline data comparable for research on metabolic disease such as obesity, diabetes and hyperlipidemia, and the like are accumulated a lot, and therefore it is suitable for the pharmacological effect test of this example, and thus this model was selected.

The mouse model that was fed the obesity feed for 8 weeks was subjected to a 2-week inspection and purification period, and during this period, the general symptoms were observed once a day to check the health and whether it was suitable for conducting the experiment, to select healthy animals. During the purification period, the individual was marked with a red oil pen on the tail of the animal at the time of acquisition (tail marking), and in the breeding box, a temporary individual identification card (test name, individual number and arrival time) was attached during the inspection and purification period. When separating the group, the individual was marked with a black oil pen on the tail of the animal, and an individual identification card (test name, group information, individual number, gender, arrival time, administration period) was attached to each cage.

To minimize stress of the experimental animal by subcutaneous administration of the test material (fusion polypeptide), sterile distilled physiological saline was subcutaneously administered at 200 uL/head to all animals using a 1 ml syringe 3 days before administration of the test material, to perform pre-adaptation training for subcutaneous administration.

For healthy animals with no abnormalities found during the inspection and purification period, the weight and feed intake were measured for all individuals after completion of the purification period.

The weight and feed intake were measured and group separation was performed so that the mean of two measurements between groups was similar based on the body weight. The administration of the test material was initiated the day after the group separation. Residual animals not selected were excluded from the test system after the end of the group separation.

The information of the high fat diet (obesity feed; High fat diet (HFD)) fed to the C57BL/6J -DIO was as follows: 5.24 kcal/g, fat 60 % by weight, protein 20 % by weight, and carbohydrate-derived calories 20 % by weight; Research Diet Inc., U.S.A.; Product No. High fat diet (Fat 60 kcal%, D12492).

The feed was fed in a free feeding (feeding during the purification and test period) manner.

As the drinking manner, the tap water was filtered with a filter water flowing sterilizer and then irradiated with ultraviolet light, and it was freely consumed using a polycarbonate drinking water bottle (250 mL).

**2.1.1. HIgG1-GDF (CRL) and IgG1-GDF(CRL)**

The administration of the test materials (HIgG1-GDF(CRL) and IgG1-GDF(CRL)) and control material GDF15 (R&D Systems) was conducted the day after the group separation, and the administration time was 9 AM every day. All the control material and test materials were administered subcutaneously. The administration route of the control material and test materials was selected as a subcutaneous route depending on the clinical intended route of administration.

The dose of all the control material and test materials was 5 mL/kg, and the dose by individual was calculated on the basis of the recently measured weight, and they were administered subcutaneously once at the test start day using a disposable syringe (1 mL). The test materials were administered only once at the test start day. For comparison, the control group in which the control material GDF15 was prepared, and for the comparison group in which GDF15 was administered, they were administered once a day for 5 days, 5 times in total, and all administrations were progressed from 9 AM

The test group composition and administration dose, and the like were summarized in the following Table 6:

[Table 6]

| IgG1-GDF (CRL) fusion polypeptide administration group composition | | | | | |
|---|---|---|---|---|---|
| High Fat Diet | Test material | Administrat ion route | Administ ration dose (nmol/kg ) | Administrat ion volume (mL/kg) | Animal number |
| X | Vehicle | Subcutaneo us | - | 5 | 5 |
| O | Vehicle, qd | Subcutaneo us | - | 5 | 5 |
| O | Vehicle, qw | Subcutaneo us | - | 5 | 5 |
| O | GDF15 | Subcutaneo us | 2.86 | 5 | 3 |
| O | IgG1-GDF (CRL) | Subcutaneo us | 1 | 5 | 5 |
| O | IgG1-GDF (CRL) | Subcutaneo us | 10 | 5 | 5 |
| O | HIgG1-GDF (CRL) | Subcutaneo us | 1 | 5 | 5 |
| O | HIgG1-GDF (CRL) | Subcutaneo us | 10 | 5 | 5 |

The observation, measurement and examination schedule for the test group was set Day 0 for the start of administration, and 7 days from the start of administration was set as 1 week of administration.

The examination schedule was summarized in Table 7:

【Table 7】

Examination schedule

| Observation item | Purification period (week) | | Period (day) | | | | | | | |
|---|---|---|---|---|---|---|---|---|---|---|
| | 1 | 2 | 0 | 1 | 2 | 3 | 4 | 5 | 6 | 7 |
| High fat feed feeding | ● | ● | ● | ● | ● | ● | ● | ● | ● | ● |
| Oral and subcutaneous administration adaptation | | ● | | | | | | | | |
| Administration | | | ● | | | | | | | |
| Weight measurement | | ● | ● | ● | ● | ● | ● | ● | ● | ● |
| Feed intake measurement | | | ● | ● | ● | ● | ● | ● | ● | ● |

For all animals, general clinical symptoms were observed once a day, and the presence of dying and dead animals was confirmed twice a day, and this observation was conducted from 1 day of administration to the end of administration. During the observation, only the case that there was an abnormal symptom was recorded on a recording sheet.

The weight of each mouse was measured at the day of the start of administration of the test materials (before administration), and then the weight was measured every day (measured by 9 days at maximum). The dose of the test materials was determined on the basis of the most recently measured weight.

In addition, after administering the test materials to mice, the feed intake was measured every day, and the amount of feeding was measured using an electronic scale by each breeding box, and then the residual amount was measured to calculate the feed intake a day. Individuals who severely ate the feed were excluded from the measurement.

All the experimental results obtained in this example were represented by mean ± standard deviation and were examined using Prism5 (version 5.01). For all the data, one-way analysis of variance (ANOVA) was performed, and when the significance was observed, Dunnett's test was conducted to find the test group with a significant difference with the control group (significance level: both sides 5% and 1%, 0.1%).

**2.1.2. IgG4-GDF and IgG4-GDF (CRL)**

The test materials (IgG4-GDF and IgG4-GDF(CRL)) and control material Semaglutide (Bachem) were subcutaneously administered. The dose of all the control material and test materials was 5 mL/kg, and the dose by individual was calculated on the basis of the recently measured weight, and they were administered subcutaneously once at the test start day using a disposable syringe (1 mL). The test materials were administered only once at the test start day, and for comparison, the control group which is administered with the control material Semaglutide was prepared. For a comparison group in which Semaglutide was administered, Semaglutide were administered every day once a day. All administrations were progressed from 9 AM.

The test group composition and administration dose, and the like were summarized in the following Table 8:

[Table 8]

| IgG4-GDF15 fusion polypeptide administration group composition | | | | | |
|---|---|---|---|---|---|
| High Fat Diet | Test material | Administrati on route | Administ ration dose (nmol/kg ) | Administ ration volume (mL/kg) | Anim al numb er |
| O | Vehicle, qw | Subcutaneo us | - | 5 | 5 |
| O | Semaglutide, qd | Subcutaneo us | 3 | 5 | 5 |
| O | IgG4-GDF | Subcutaneo us | 1 | 5 | 5 |
| O | IgG4-GDF | Subcutaneo us | 10 | 5 | 5 |
| O | IgG4-GDF (CRL) | Subcutaneo us | 1 | 5 | 5 |
| O | IgG4-GDF (CRL) | Subcutaneo us | 10 | 5 | 5 |

The observation, measurement and examination schedule for the test groups was set Day 0 for the start of administration, and they were conducted as same as 2.1.1.

The weight of each mouse was measured at the day of the start of administration of the test materials (before administration), and then the weight was measured every day (measured by 19 days at maximum). The dose of the test materials was determined on the basis of the most recently measured weight.

In addition, after administering the test materials to mice, the feed intake was measured every day, and the amount of feeding was measured using an electronic scale by each breeding box, and then the residual amount was measured to calculate the feed intake a day. Individuals who severely ate the feed were excluded from the measurement.

### 2.2. Weight loss test result

#### 2.2.1. HIgG1-GDF (CRL) and IgG1-GDF(CRL)

The weight change measured in the Example 2.1.1 was shown in FIG. 5 and FIG. 6, and Table 9 (Body Weight (Group, % of initial)).

[Table 9]

| Group | Day | 0 | 1 | 2 | 3 | 4 | 5 | 6 | 7 | 8 | 9 |
|---|---|---|---|---|---|---|---|---|---|---|---|
| Lean Vehicle Control (Daily inj.) | Mea n | 100 | 102 | 101 | 101 | 101 | 101 | 101 | 101 | 101 | 102 |
| | S.E. | 0 | 1 | 1 | 1 | 1 | 1 | 1 | 1 | 1 | 1 |
| DIO Vehicle Control (Daily inj.) | Mea n | 100 | 100 | 100 | 100 | 100 | 100 | 101 | 101 | 101 | 101 |
| | S.E. | 0 | 0 | 0 | 0 | 1 | 1 | 1 | 1 | 0 | 0 |
| DIO Vehicle Control (Single Inj.) | Mea n | 100 | 100 | 99 | 100 | 100 | 100 | 101 | 101 | 102 | 102 |
| | S.E. | 0 | 0 | 0 | 0 | 1 | 1 | 1 | 1 | 1 | 1 |
| GDF15, 2.86 nmol/kg (Daily Inj.) | Mea n | 100 | 99 | 98 | 97 | 96 | 95 | 95 | 95 | 96 | 97 |
| | S.E. | 0 | 0 | 0 | 1 | 1 | 1 | 1 | 2 | 2 | 2 |
| IgG1-GDF(CRL), 1 nmol/kg (Single Inj.) | Mea n | 100 | 99 | 98 | 96 | 96 | 96 | 95 | 95 | 95 | 95 |
| | S.E. | 0 | 1 | 1 | 0 | 1 | 1 | 1 | 1 | 1 | 1 |
| IgG1-GDF(CRL), 10 nmol/kg (Single Inj.) | Mea n | 100 | 98 | 97 | 95 | 94 | 93 | 91 | 90 | 90 | 90 |
| | S.E. | 0 | 0 | 1 | 1 | 1 | 1 | 1 | 1 | 1 | 1 |
| HIgG1-GDF(CRL), 1 nmol/kg (Single Inj.) | Mea n | 100 | 100 | 98 | 98 | 97 | 96 | 97 | 96 | 96 | 97 |
| | S.E. | 0 | 0 | 0 | 0 | 0 | 0 | 0 | 0 | 0 | 1 |
| HIgG1-GDF(CRL), 10 nmol/kg (Single Inj.) | Mea n | 100 | 99 | 98 | 97 | 95 | 95 | 94 | 93 | 93 | 93 |
| | S.E. | 0 | 0 | 0 | 0 | 1 | 1 | 1 | 1 | 1 | 1 |

(In the Table 9, Day0: first administration of control material GDF15, single administration of IgG1-GDF (CRL) fusion polypeptide; Lean: group without high fat diet)

FIG. 5 and Table 9 show the weight change after single administration of fusion protein IgG1-GDF by comparing with the negative control group (vehicle administration group) and positive control group (GDF15 daily administration group). In addition, FIG. 6 is a graph extracting and showing the result at 7 days among the results of FIG. 5.

As shown in the results, it can be confirmed that there was little weight change in case of the negative control group (vehicle administration group), while the weight loss effect disappeared from Day 6, the first day after stopping administration in case of the GDF15 daily administration group (stopping administration from Day 5). On the other hand, it can be confirmed that the weight loss effect was shown immediately after single administration at Day 0 in case of the fusion polypeptide in which GDF15(CRL) was fused with IgG1 or Fc comprising Hinge, and the weight loss effect was not reduced and was shown consistently throughout the test period (9 days), and as time went by, the concentration weight loss effect was increased, and the weight loss effect was concentration-dependent. This weight loss effect of the fusion polypeptide can be said to be comparable when GDF15 is administered once a day throughout the test period.

#### 2.2.2. IgG4-GDF and IgG4-GDF(CRL)

The weight change measured in Example 2.1.2 was shown in FIG. 7 and Table 10 (Body Weight (Group, % of initial)).

[Table 10]

| Group | Day | 0 | 1 | 2 | 3 | 4 | 5 | 6 | 7 | 8 | 9 |
|---|---|---|---|---|---|---|---|---|---|---|---|
| DIO Vehicle Control (Single Inj.) | Mea n | 10 0 | 10 0 | 10 0 | 99 | 10 0 | 10 0 | 10 0 | 10 0 | 10 1 | 10 1 |
| | S.E. | 0 | 0 | 1 | 0 | 0 | 0 | 0 | 1 | 0 | 1 |
| Semaglutide, 3 nmol/kg (Daily Inj.) | Mea n | 10 0 | 94 | 91 | 90 | 87 | 87 | 85 | 86 | 83 | 85 |
| | S.E. | 0 | 0 | 1 | 2 | 2 | 3 | 3 | 3 | 3 | 3 |
| IgG4-GDF15, 1 nmol/kg (Single Inj.) | Mea n | 10 0 | 99 | 97 | 96 | 95 | 93 | 93 | 92 | 92 | **91** |
| | S.E. | 0 | 0 | 1 | 1 | 1 | 1 | 1 | 1 | 1 | **1** |
| IgG4-GDF15, 10 nmol/kg (Single Inj.) | Mea n | 10 0 | 98 | 97 | 96 | 94 | 93 | 93 | 92 | 91 | **90** |
| | S.E. | 0 | 0 | 1 | 1 | 1 | 1 | 1 | 1 | 2 | **2** |
| IgG4-GDF(CRL), 1 nmol/kg (Single Inj.) | Mea n | 10 0 | 99 | 98 | 97 | 96 | 95 | 94 | 93 | 92 | 92 |
| | S.E. | 0 | 0 | 0 | 0 | 1 | 1 | 1 | 1 | 1 | 1 |
| IgG4-GDF(CRL), 10 nmol/kg (Single Inj.) | Mea n | 10 0 | 98 | 97 | 95 | 94 | 92 | 91 | 90 | 88 | 87 |
| | S.E. | 0 | 0 | 0 | 1 | 1 | 1 | 1 | 1 | 1 | 1 |
| **Group** | **Day** | **10** | **11** | **12** | **13** | **14** | **15** | **16** | **17** | **18** | **19** |
| DIO Vehicle Control (Single Inj.) | Mea n | 10 2 | 10 2 | 10 3 | 10 4 | 10 4 | 104 | 10 5 | 10 5 | 10 5 | **10 5** |
| | S.E. | 1 | 1 | 0 | 1 | 1 | 1 | 1 | 1 | 1 | **1** |
| Semaglutide, 3 nmol/kg (Daily Inj.) | Mea n | 84 | 82 | 81 | 82 | 81 | 79 | 79 | 78 | 77 | **77** |
| | S.E. | 3 | 3 | 3 | 3 | 3 | 2 | 2 | 2 | 2 | **1** |
| IgG4-GDF15, 1 nmol/kg (Single Inj.) | Mea n | 91 | 91 | 93 | 94 | 94 | 94 | 95 | 96 | 96 | **96** |
| | S.E. | 1 | 1 | 1 | 1 | 1 | 1 | 1 | 2 | 1 | **2** |
| IgG4-GDF15, 10 nmol/kg (Single Inj.) | Mea n | 90 | 90 | 91 | 92 | 92 | 92 | 92 | 93 | 94 | **94** |
| | S.E. | 2 | 1 | 2 | 2 | 2 | 2 | 2 | 2 | 2 | **2** |
| IgG4-GDF(CRL), 1 nmol/kg (Single Inj.) | Mea n | **91** | 91 | 91 | 91 | 91 | 91 | 91 | 92 | 92 | **92** |
| | S.E. | **1** | 1 | 1 | 1 | 1 | 1 | 1 | 2 | 2 | **2** |
| IgG4-GDF(CRL), | Mea | 85 | 85 | 84 | 83 | 83 | **82** | 83 | 84 | 84 | **85** |
| 10 nmol/kg (Single Inj.) | n | | | | | | | | | | |
| | S.E. | 1 | 1 | 1 | 1 | 2 | **2** | 2 | 2 | 3 | **2** |

(In the Table 10, Day0: first administration of control material (Semaglutide) and test materials; The control material was administered every day; The test materials were administered once)

FIG. 7 and FIG. 8, and Table 10 show the weight change after single administration of fusion protein IgG4-GDF and IgG4-GDF (CRL) by comparing with the negative control group (vehicle administration group) and positive control group (Semaglutide daily administration group). In addition, FIG. 8 is a graph extracting and showing the result at 7 days and 14 days among the results of FIG. 7.

As shown in the results, it can be confirmed that the weight was slightly increased in case of the negative control group (vehicle administration group), while the weight loss was consistent in case of the positive control group (Semaglutide daily administration group). When IgG4-GDF15 was administered once, regardless of dose, the weight loss effect lasted up to 9 days. On the other hand, it can be confirmed that the weight loss effect was shown consistently up to 10 days when 1 nmol/kg of IgG4-GDF(CRL) was administered once and up to 15 days when 10 nmol/kg was administered once, and as time went by, the concentration weight loss effect was increased, and the weight loss effect was concentration-dependent. In addition, it was confirmed that the GDF (CRL) fusion polypeptide with Fc (Mutated) of IgG4 without Hinge had the excellent weight loss effect at the same dose, compared to the GDF (CRL) fusion polypeptide with Fc (Mutated) of IgG4 comprising Hinge. The weight loss effect of this IgG4 Fc fusion polypeptide can be said to be comparable when Semaglutide is administered once a day throughout the test period.

### 2.3. Diet intake test result

#### 2.3.1. HIgG1-GDF (CRL) and IgG1-GDF(CRL)

The feed intake change measured in Example 2.1.1 was shown in Table 11 and FIG. 9 (cumulative intake by 6 days), respectively.

[Table 11]

| Group | Day | 0 | 1 | 2 | 3 | 4 | 5 | 6 | 7 | 8 | 9 | |
|---|---|---|---|---|---|---|---|---|---|---|---|---|
| Lean Vehicle Control (Daily inj.) | Mea n | 4.5 | 4.6 | 4.6 | 4.3 | 4.4 | 4.6 | 4.2 | 4.5 | 3.9 | 4.7 | |
| | S.E. | 0.2 | 0.2 | 0.1 | 0.2 | 0.1 | 0.3 | 0.2 | 0.2 | 0.1 | 0.3 | |
| DIO Vehicle Control (Daily inj.) | Mea n | 2.8 | 2.8 | 3.1 | 3.0 | 3.1 | 2.9 | 3.0 | 2.9 | 2.9 | 3.3 | |
| | S.E. | 0.3 | 0.2 | 0.2 | 0.3 | 0.3 | 0.2 | 0.3 | 0.2 | 0.1 | 0.2 | |
| DIO Vehicle Control (Single Inj.) | Mea n | 3.1 | 2.9 | 3.1 | 3.2 | 3.3 | 3.2 | 3.3 | 3.2 | 3.0 | 3.5 | |
| | S.E. | 0.1 | 0.2 | 0.2 | 0.2 | 0.2 | 0.1 | 0.1 | 0.1 | 0.1 | 0.2 | |
| GDF15, 2.86 nmol/kg (Daily Inj.) | Mea n | 2.8 | 2.4 | 2.7 | 2.4 | 2.5 | 2.6 | 2.7 | 3.0 | 3.3 | 3.5 | |
| | S.E. | 0.4 | 0.3 | 0.2 | 0.4 | 0.2 | 0.3 | 0.2 | 0.3 | 0.2 | 0.3 | |
| IgG1-GDF(CRL), 1 nmol/kg (Single Inj.) | Mea n | 3.0 | 2.1 | 2.2 | 2.2 | 2.6 | 2.6 | 2.6 | 2.8 | 2.7 | 3.0 | |
| | S.E. | 0.2 | 0.2 | 0.1 | 0.1 | 0.2 | 0.1 | 0.0 | 0.1 | 0.1 | 0.3 | |
| IgG1-GDF(CRL), 10 nmol/kg (Single Inj.) | Mea n | 2.7 | 2.0 | 2.5 | 2.0 | 1.9 | 1.9 | 2.1 | 2.1 | 2.3 | 2.8 | |
| | S.E. | 0.3 | 0.1 | 0.1 | 0.3 | 0.2 | 0.2 | 0.2 | 0.1 | 0.1 | 0.3 | |
| HIgG1-GDF(CRL), 1 nmol/kg (Single Inj.) | Mea n | 3.0 | 2.4 | 2.4 | 2.5 | 2.7 | 2.6 | 2.9 | 2.7 | 2.9 | 3.0 | |
| | S.E. | 0.2 | 0.1 | 0.1 | 0.0 | 0.1 | 0.0 | 0.1 | 0.1 | 0.1 | 0.1 | |
| HIgG1-GDF(CRL), 10 nmol/kg (Single Inj.) | Mea n | 3.8 | 2.2 | 2.2 | 2.3 | 2.4 | 2.4 | 2.3 | 2.4 | 2.9 | 3.3 | |
| | S.E. | 0.5 | 0.1 | 0.1 | 0.1 | 0.2 | 0.1 | 0.1 | 0.1 | 0.3 | 0.3 | |

As shown in the result, it can be confirmed that the administration group of the fusion polypeptide in which GDF (CRL) was fused with IgG1 Fc comprising Hinge or Fc of IgG1 not comprising Hinge showed the feed intake reducing effect during the test period (9 days), compared to the negative control administration group (vehicle administration group), and the feed intake reducing effect was concentration-dependent. This feed intake reducing effect of the fusion polypeptide can be said to be comparable when GDF15 is administered once a day throughout the test period.

#### 2.3.2. IgG4-GDF and IgG4-GDF(CRL)

The feed intake change measured in Example 2.1.2 was shown in Table 12 and FIG. 10 (cumulative intake by 7 days, at 14 days and 19 days).

[Table 12]

| Group | Day | 0 | 1 | 2 | 3 | 4 | 5 | 6 | 7 | 8 | 9 |
|---|---|---|---|---|---|---|---|---|---|---|---|
| DIO Vehicle Control (Single Inj.) | Mea n | 3.3 | 2.7 | 3.0 | 3.0 | 3.1 | 3.5 | 3.0 | 3.5 | 3.3 | 3.4 |
| | S.E. | 0.1 | 0.1 | 0.2 | 0.1 | 0.1 | 0.2 | 0.2 | 0.1 | 0.1 | 0.2 |
| Semaglutide, 3 nmol/kg (Daily Inj.) | Mea n | 3.7 | 2.1 | 3.3 | 2.8 | 2.3 | 2.9 | 2.7 | 3.2 | 2.4 | 3.7 |
| | S.E. | 0.3 | 0.7 | 1.4 | 0.6 | 0.4 | 0.9 | 0.6 | 0.3 | 0.4 | 0.1 |

(continued)

| Group | Day | 0 | 1 | 2 | 3 | 4 | 5 | 6 | 7 | 8 | 9 |
|---|---|---|---|---|---|---|---|---|---|---|---|
| IgG4-GDF15, 1 nmol/kg (Single Inj.) | Mea n | 3.0 | 1.9 | 1.9 | 2.1 | 2.0 | 2.0 | 2.1 | 2.4 | 2.4 | 2.5 |
| | S.E. | 0.1 | 0.1 | 0.1 | 0.2 | 0.2 | 0.1 | 0.1 | 0.2 | 0.2 | 0.2 |
| IgG4-GDF15, 10 nmol/kg (Single Inj.) | Mea n | 3.0 | 1.7 | 2.0 | 2.0 | 2.1 | 2.2 | 2.4 | 2.4 | 2.2 | 2.7 |
| | S.E. | 0.1 | 0.1 | 0.3 | 0.2 | 0.2 | 0.3 | 0.2 | 0.2 | 0.2 | 0.2 |
| IgG4-GDF(CRL), 1 nmol/kg (Single Inj.) | Mea n | 3.3 | 2.0 | 2.6 | 2.7 | 2.4 | 2.7 | 2.3 | 2.5 | 2.5 | 2.8 |
| | S.E. | 0.2 | 0.1 | 0.2 | 0.2 | 0.1 | 0.1 | 0.2 | 0.1 | 0.2 | 0.1 |
| IgG4-GDF(CRL), 10 nmol/kg (Single Inj.) | Mea n | 3.1 | 1.5 | 2.3 | 2.4 | 2.4 | 2.3 | 2.3 | 2.0 | 2.3 | 2.4 |
| | S.E. | 0.2 | 0.3 | 0.2 | 0.2 | 0.2 | 0.2 | 0.2 | 0.1 | 0.2 | 0.3 |
| **Group** | **Day** | **10** | **11** | **12** | **13** | **14** | **15** | **16** | **17** | **18** | **19** |
| DIO Vehicle Control (Single Inj.) | Mea n | 3.0 | 3.4 | 3.2 | 3.5 | 3.1 | 3.3 | 3.1 | 3.2 | 3.3 | 3.3 |
| | S.E. | 0.2 | 0.1 | 0.2 | 0.1 | 0.1 | 0.2 | 0.1 | 0.1 | 0.1 | 0.1 |
| Semaglutide, 3 nmol/kg (Daily Inj.) | Mea n | 2.7 | 2.5 | 2.4 | 3.0 | 2.5 | 2.1 | 2.5 | 2.0 | 2.2 | 3.7 |
| | S.E. | 0.4 | 0.4 | 0.3 | 0.4 | 0.3 | 0.3 | 0.2 | 0.2 | 0.2 | 0.3 |
| IgG4-GDF15, 1 nmol/kg (Single Inj.) | Mea n | 2.7 | 3.1 | 3.2 | 3.7 | 3.0 | 3.1 | 3.1 | 3.1 | 3.1 | 3.0 |
| | S.E. | 0.1 | 0.0 | 0.0 | 0.1 | 0.1 | 0.3 | 0.2 | 0.3 | 0.1 | 0.1 |
| IgG4-GDF15, 10 nmol/kg (Single Inj.) | Mea n | 3.1 | 3.8 | 3.3 | 3.7 | 3.4 | 3.6 | 3.1 | 3.5 | 3.3 | 3.0 |
| | S.E. | 0.3 | 0.7 | 0.5 | 0.5 | 0.4 | 0.6 | 0.2 | 0.1 | 0.1 | 0.1 |
| IgG4-GDF(CRL), 1 nmol/kg (Single Inj.) | Mea n | 2.4 | 2.8 | 2.6 | 3.0 | 2.8 | 2.9 | 3.1 | 3.3 | 3.0 | 3.3 |
| | S.E. | 0.1 | 0.1 | 0.2 | 0.2 | 0.2 | 0.2 | 0.2 | 0.2 | 0.2 | 0.2 |
| IgG4-GDF(CRL), 10 nmol/kg (Single Inj.) | Mea n | 2.1 | 2.5 | 2.3 | 2.5 | 2.6 | 2.6 | 3.0 | 3.4 | 3.3 | 3.1 |
| | S.E. | 0.1 | 0.2 | 0.3 | 0.2 | 0.4 | 0.2 | 0.3 | 0.3 | 0.3 | 0.2 |

As shown in the result, it can be confirmed that the administration group of the fusion polypeptide in which GDF15 or GDF (CRL) was fused with IgG4 (mutated) not comprising Hinge showed the feed intake reducing effect during the test period (19 days), compared to the negative control administration group (vehicle administration group). This feed intake reducing effect of the fusion polypeptide can be said to be comparable when Semaglutide is administered once a day throughout the test period. When comparing administration groups of 10 nmol/kg of the fusion polypeptide in which GDF15 or GDF (CRL) was fused with IgG4 Fc (Mutated) not comprising Hinge, in case of the administration group of the full-length GDF15-fused fusion polypeptide, the diet suppression ability was maintained for about 10 days, and in case of the administration group of the GDF(CRL)-fused fusion polypeptide, the diet suppression ability was maintained for about 2 weeks. In other words, it was confirmed that the GDF (CRL) fusion polypeptide had a little excellent weight loss effect at 10 nmol/kg dose, compared to the full-length GDF15 fusion polypeptide.

**Example 3. Pharmacodynamic test of fusion polypeptide (IgG4-GDF or IgG4-GDF(CRL))**

**3.1. Preparation of test group and control group serum**

For evaluation of pharmacodynamic characteristics when each polypeptide was subcutaneously administered to rats, polypeptide IgG4-GDF or IgG4-GDF(CRL) was subcutaneously administered in an amount of 2 mg/kg, respectively, to SD Rat (Koatech, male, 7 weeks, about 250g; n=3 each; test group), and at a fixed time, blood was collected about 200μl through a caudal vein. Blood collecting was progressed before administration of the fusion polypeptide, in 1, 2, 4, 8, 24, 48, 72, 96, 168, 240 and 336 hours after administration. As a control group for comparison of pharmacodynamic characteristics, GDF15 (R&D Systems) was subcutaneously administered in an amount of 2 mg/kg by the same method as above to prepare GDF15 administration group.

After administering to SD Rat as above, blood collected by time-point was centrifuged to obtain serum, and ELISA was performed using Human GDF15 Immunoassay (SGD150, R&D Systems), to measure the concentration in serum over time for each polypeptide. Using these data, the values of parameters including AUC (area under the curve) was obtained using a software for PK analysis (WinNonlin (Certara L.P.) et al.).

### 3.2 Pharmacodynamic test result

The obtained pharmacodynamic parameters of the fusion polypeptide were shown in Table 13, and the concentration change of the fusion polypeptide over time was shown in FIG. 11:

[Table 13]

| PK Parameter | Group 1 | Group 2 | Group 3 |
|---|---|---|---|
| | GDF15 | IgG4-GDF | IgG4-GDF(CRL) |
| $C_{max}$ (ug/mL) | 0.443 | 10.6 | 9.73 |
| $T_{max}$ (hr) | 1 | 96 | 48 |
| $AUC_{last}$ (ug·hr/mL) | 4.86 | 2144 | 2004 |
| $AUC_{inf}$ (ug·hr/mL) | 4.88 | 2854 | 2593 |
| $t_{1/2}$ (hr) | 19.0 | 101 | 114 |
| $AUC_{extp}$ (%) | 0.463 | 14.6 | 18.2 |
| ($C_{max}$: maximum blood concentration, $T_{max}$: Time to reach maximum blood concentration, $AUC_{inf}$: area under the blood concentration-time curve calculated by extrapolating from the last measurable blood collection time to infinite time, $AUC_{last}$: area under the blood concentration-time curve by the last measurable blood collection time, $T_{1/2}$: elimination half-life, $AUC_{Extp}$(%): [($AUC_{inf}$-$AUC_{las}$t)/$AUC_{inf}$]* 100) | | | |

As shown in the result, it can be confirmed that compared with GDF15 (half-life: 19 hours), in case of IgG4-GDF15 (half-life: 101 hours)and IgG4-GDF (CRL) (half-life: 114 hours) fusion proteins, the stability in blood (serum) was significantly increased (5 times or more).

From the above description, those skilled in the art to which the present invention belongs will appreciate that the present invention may be implemented in other specific forms without changing its technical spirit or essential features. In this regard, the embodiments described above should be understood as illustrative and not restrictive in all aspects. The scope of the present invention should be construed to include all the altered or modified forms derived from the meaning and scope of the claims which will be described later, and their equivalent concepts, rather than the above detailed description.

## Claims

1. A fusion polypeptide, comprising

GDF15 (Growth/differentiation factor 15) or its functional variant, and
Fc region of an immunoglobulin,
Wherein,
the Fc region of the immunoglobulin is a single chain of IgG1 Fc region or IgG4 Fc region, and is linked to the N-terminus of the GDF15 or its functional variant, via a flexible peptide linker,
the functional variant of GDF15 is a deletion variant in which at least one of 14 amino acids at positions 1 to 14 of the amino acid sequence of SEQ ID NO: 1 are deleted, and
the flexible peptide linker is represented by (GGGGS)n (n is 1, 2, 3, 4, or 5).

**2.** The fusion polypeptide according to claim 1, wherein the Fc region of the immunoglobulin is human IgG4 Fc region.

**3.** The fusion polypeptide according to claim 2, wherein the human IgG4 Fc region

(1) comprises the amino acid sequence of SEQ ID NO: 5, or
(2) further comprises the amino acid sequence of SEQ ID NO: 10 at the N-terminus of the amino acid sequence of SEQ ID NO: 5.

**4.** The fusion polypeptide according to claim 3, wherein the human IgG4 Fc region

(1) comprises an amino acid sequence selected from SEQ ID NOs: 6 to 9, or
(2) further comprises an amino acid sequence of SEQ ID NO: 11 or SEQ ID NO: 12 at the N-terminus of an amino acid sequence selected from SEQ ID NOs: 6 to 9.

**5.** The fusion polypeptide according to claim 1, wherein the functional variant of GDF15 comprises the amino acid sequence of SEQ ID NO: 2.

**6.** The fusion polypeptide according to any one of claims 1 to 5, wherein GDF15 or its functional variant linked to the Fc region of the immunoglobulin in the fusion polypeptide has at least 1.5 times increased in vivo half-life, compared to GDF15 or its functional variant which is not linked to Fc region of the immunoglobulin.

**7.** A fusion polypeptide dimer, comprising two fusion polypeptides of any one of claims 1 to 5.

**8.** A nucleic acid molecule encoding the fusion polypeptide of any one of claims 1 to 5.

**9.** A recombinant vector comprising the nucleic acid molecule of claim 8.

**10.** A recombinant cell comprising the recombinant vector of claim 9.

**11.** A method of preparation of the fusion polypeptide of claim 1, wherein the fusion polypeptide comprises GDF15 or its functional variant and an Fc region of immunoglobulin, the method comprising culturing the recombinant cell of claim 10.

**12.** A method of enhancing in vivo stability of GDF15 or its functional variant, comprising linking a single chain of IgG1 Fc region or IgG4 Fc region to the N-terminus of GDF15 or its functional variant via a flexible peptide linker, wherein the functional variant of GDF15 is a deletion variant in which at least one of 14 amino acids at positions 1 to 14 of the amino acid sequence of SEQ ID NO: 1 are deleted, and the flexible peptide linker is represented by (GGGGS)n ((SEQ ID NO: 13)n, wherein n is 1, 2, 3, 4, or 5).

**13.** The method for enhancing in vivo stability of GDF15 or its functional variant according to claim 12, wherein the Fc region of the immunoglobulin is human IgG4 Fc region.

**14.** The method for enhancing in vivo stability of GDF15 or its functional variant according to claim 13, wherein the human IgG4 Fc region

(1) comprises the amino acid sequence of SEQ ID NO: 5, or
(2) further comprises the amino acid sequence of SEQ ID NO: 10 at the N-terminus of the amino acid sequence of SEQ ID NO: 5.

**15.** The method for enhancing in vivo stability of GDF15 or its functional variant according to claim 14, wherein the human IgG4 Fc region

(1) comprises an amino acid sequence selected from SEQ ID NOs: 6 to 9, or
(2) further comprises an amino acid sequence of SEQ ID NO: 11 or SEQ ID NO: 12 at the N-terminus of an amino acid sequence selected from SEQ ID NOs: 6 to 9.

**16.** The method for enhancing in vivo stability of GDF15 or its functional variant according to claim 12, wherein the functional variant of GDF15 comprises the amino acid sequence of SEQ ID NO: 2.

**17.** The method for enhancing in vivo stability of GDF15 or its functional variant according to any one of claims 12 to 16, wherein GDF15 or its functional variant linked to the Fc region of the immunoglobulin in the fusion polypeptide has at least 1.5 times increased in vivo half-life, compared to GDF15 or its functional variant which is not linked to Fc region of the immunoglobulin.

**18.** A composition for weight loss, comprising at least one selected from the group consisting of:

the fusion polypeptide of any one of claims 1 to 5,
a fusion polypeptide dimer comprising two of the fusion polypeptides,
a nucleic acid molecule encoding the fusion polypeptide,
a recombinant vector comprising the nucleic acid molecule, and
a recombinant cell comprising the recombinant vector.

**19.** A composition for dietary control, comprising at least one selected from the group consisting of:

the fusion polypeptide of any one of claims 1 to 5,
a fusion polypeptide dimer comprising two of the fusion polypeptides,
a nucleic acid molecule encoding the fusion polypeptide,
a recombinant vector comprising the nucleic acid molecule, and
a recombinant cell comprising the recombinant vector.

**20.** A pharmaceutical composition for prevention or treatment of metabolic disease, comprising at least one selected from the group consisting of:

the fusion polypeptide of any one of claims 1 to 5,
a fusion polypeptide dimer comprising two of the fusion polypeptides,
a nucleic acid molecule encoding the fusion polypeptide,
a recombinant vector comprising the nucleic acid molecule, and
a recombinant cell comprising the recombinant vector.

**21.** The pharmaceutical composition according to claim 20, wherein the metabolic disease is obesity, diabetes, or nonalcoholic fatty liver disease.

【FIG. 1】

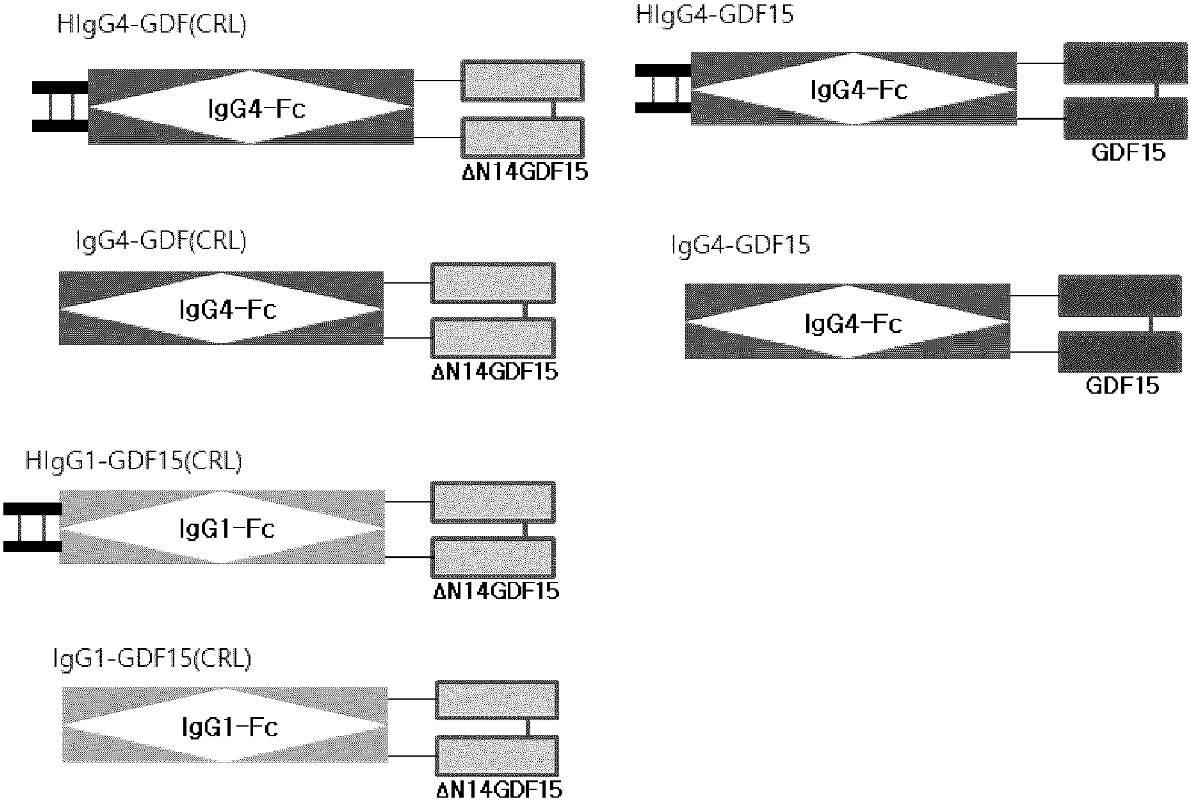


【FIG. 2】

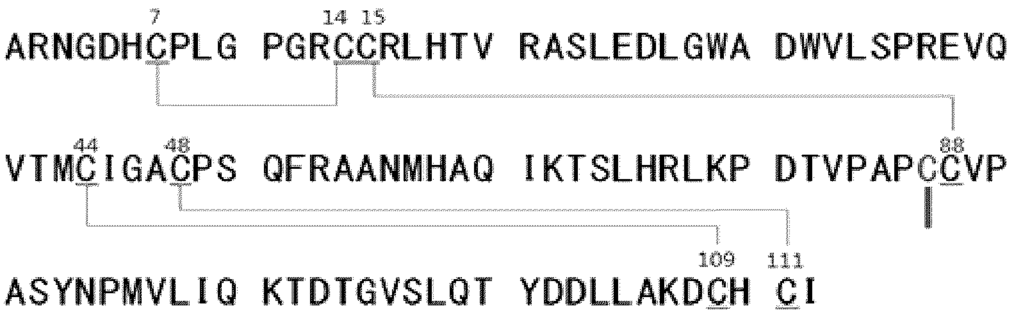


(SEQ ID NO: 1)

【FIG. 3a】

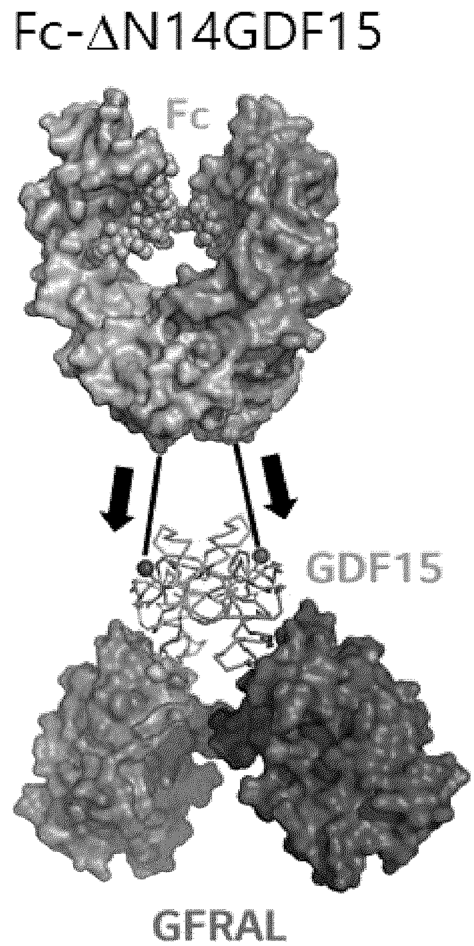

【FIG. 3b】

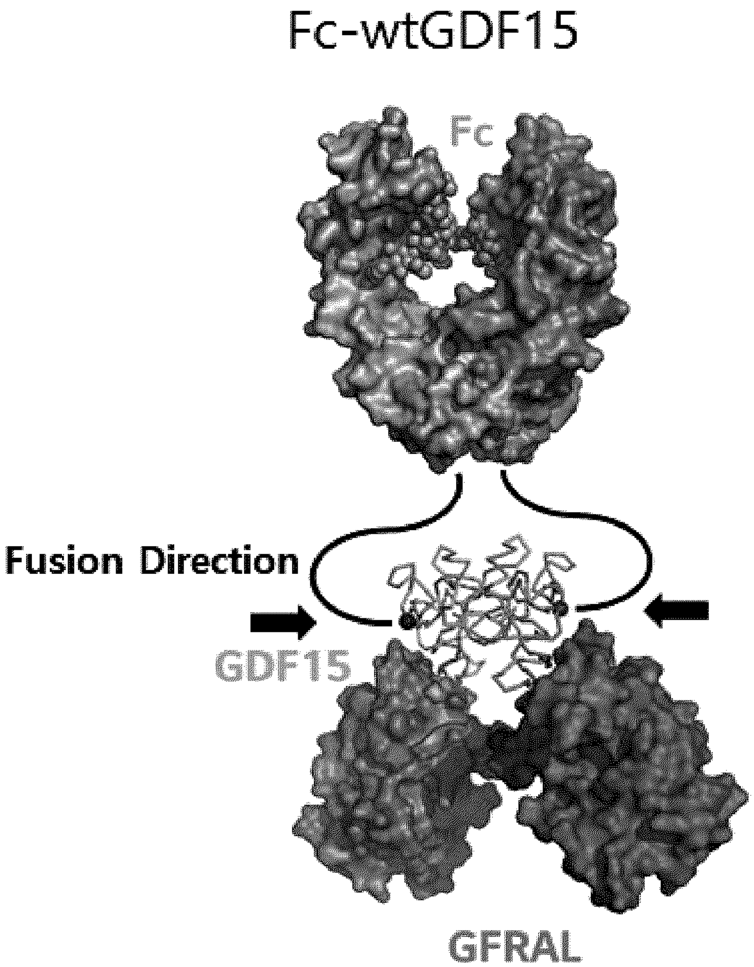

【FIG. 4】

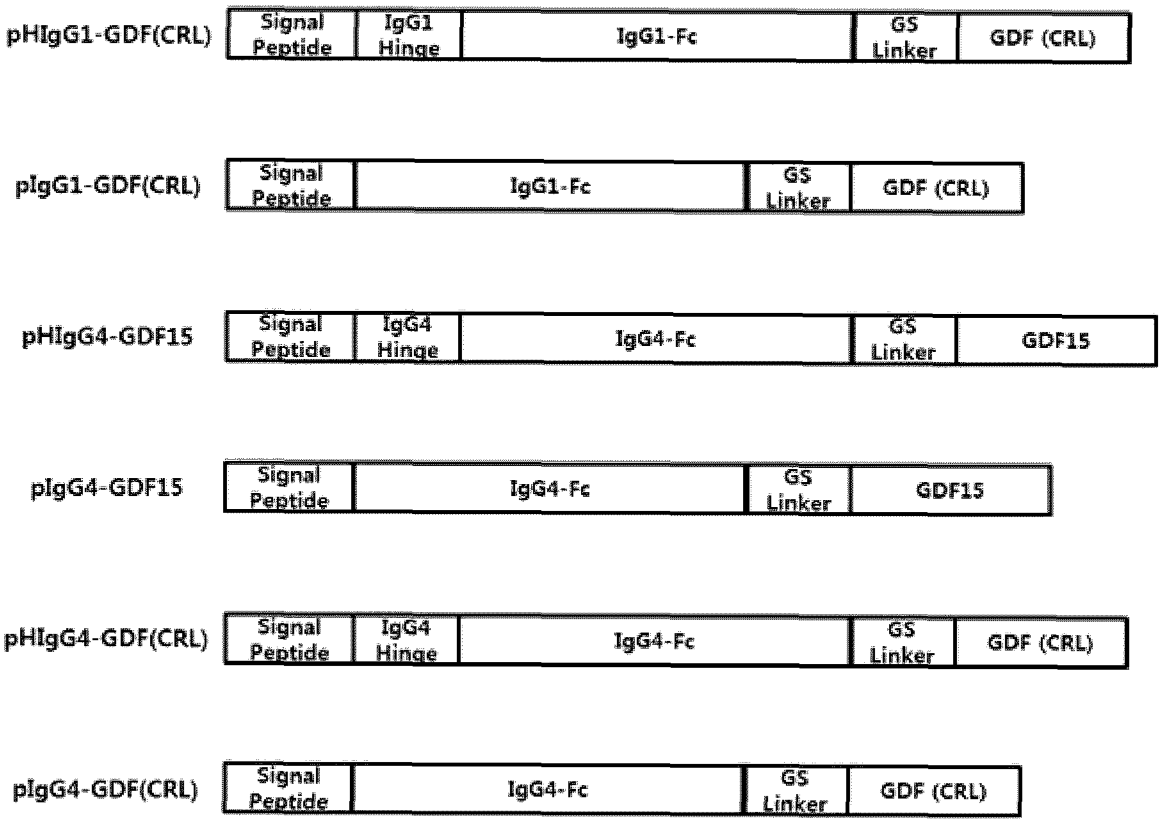
pHIgG1-GDF(CRL)
Signal Peptide
IgG1 Hinge
IgG1-Fc
GS Linker
GDF (CRL)
pIgG1-GDF(CRL)
Signal Peptide
IgG1-Fc
GS Linker
GDF (CRL)
pHIgG4-GDF15
Signal Peptide
IgG4 Hinge
IgG4-Fc
GS Linker
GDF15
pIgG4-GDF15
Signal Peptide
IgG4-Fc
GS Linker
GDF15
pHIgG4-GDF(CRL)
Signal Peptide
IgG4 Hinge
IgG4-Fc
GS Linker
GDF (CRL)
pIgG4-GDF(CRL)
Signal Peptide
IgG4-Fc
GS Linker
GDF (CRL)

【FIG. 5】

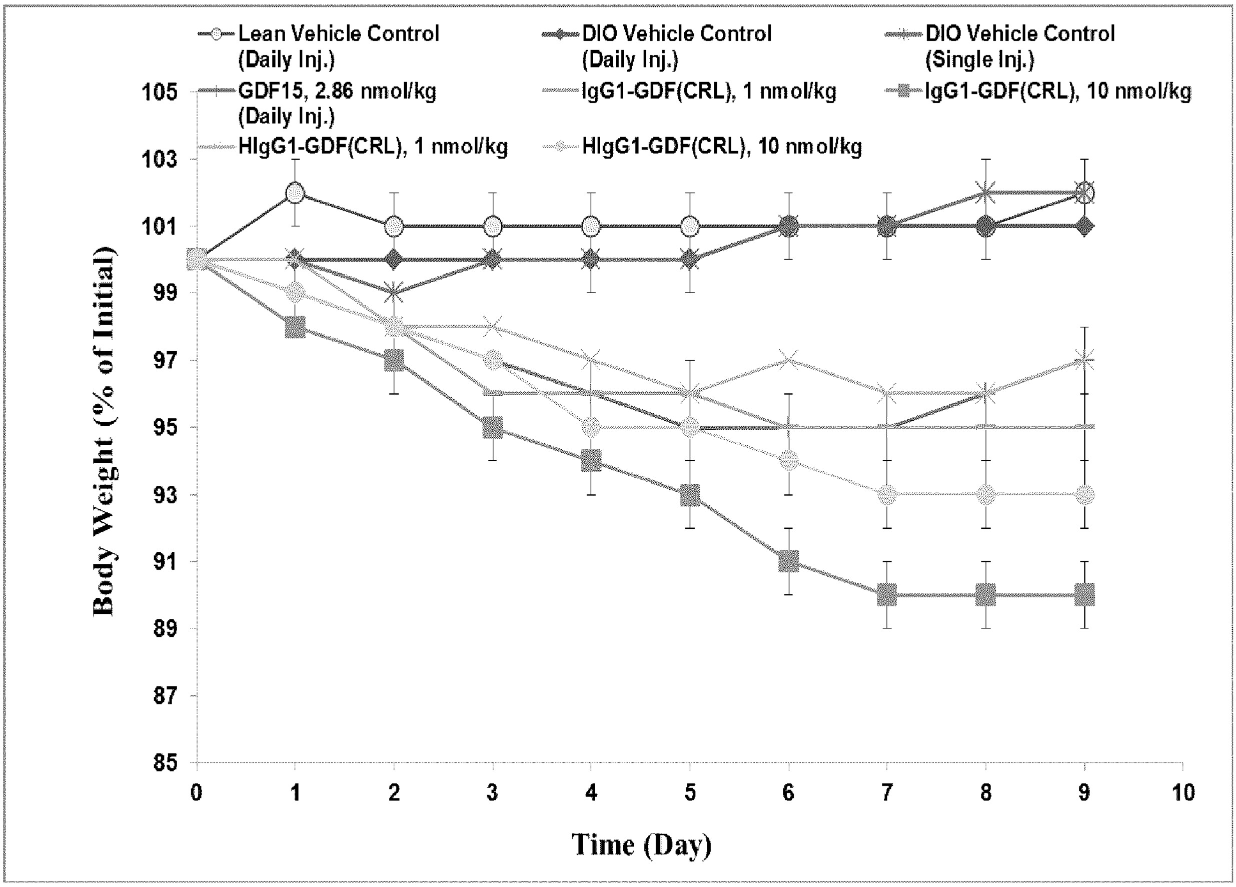
Lean Vehicle Control (Daily Inj.)
DIO Vehicle Control (Daily Inj.)
DIO Vehicle Control (Single Inj.)
GDF15, 2.86 nmol/kg (Daily Inj.)
IgG1-GDF(CRL), 1 nmol/kg
IgG1-GDF(CRL), 10 nmol/kg
HIgG1-GDF(CRL), 1 nmol/kg
HIgG1-GDF(CRL), 10 nmol/kg
105
103
101
99
97
95
93
91
89
87
85
Body Weight (% of Initial)
0 1 2 3 4 5 6 7 8 9 10
Time (Day)

【FIG. 6】

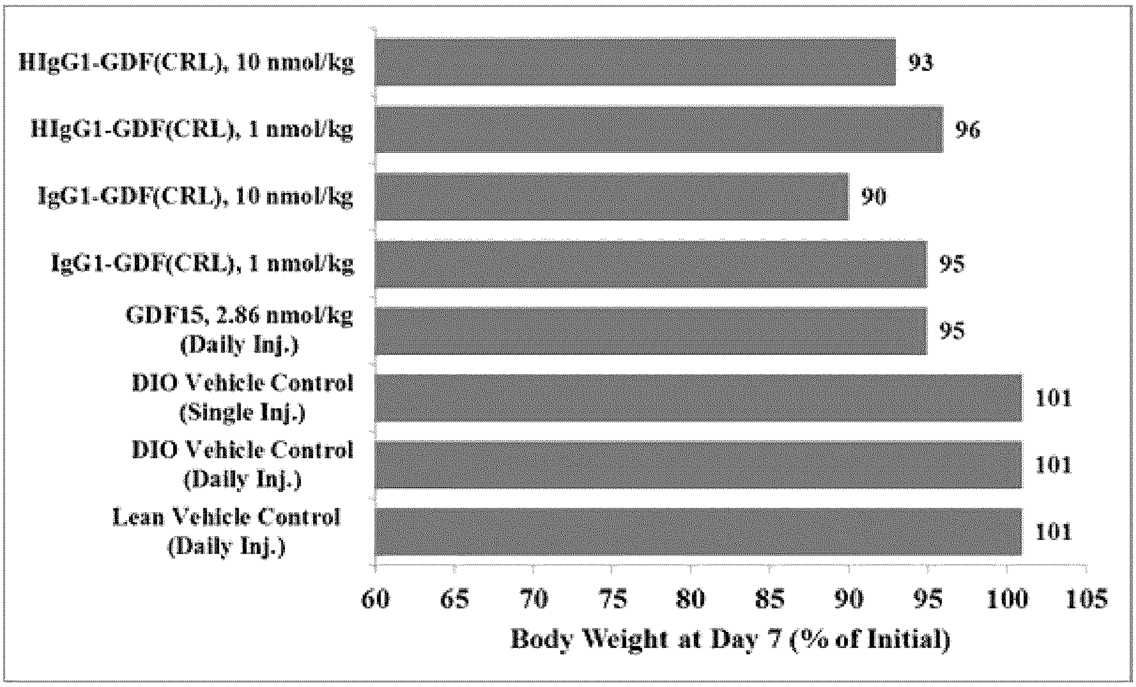
HIgG1-GDF(CRL), 10 nmol/kg
93
HIgG1-GDF(CRL), 1 nmol/kg
96
IgG1-GDF(CRL), 10 nmol/kg
90
IgG1-GDF(CRL), 1 nmol/kg
95
GDF15, 2.86 nmol/kg (Daily Inj.)
95
DIO Vehicle Control (Single Inj.)
101
DIO Vehicle Control (Daily Inj.)
101
Lean Vehicle Control (Daily Inj.)
101
60 65 70 75 80 85 90 95 100 105
Body Weight at Day 7 (% of Initial)

【FIG. 7】

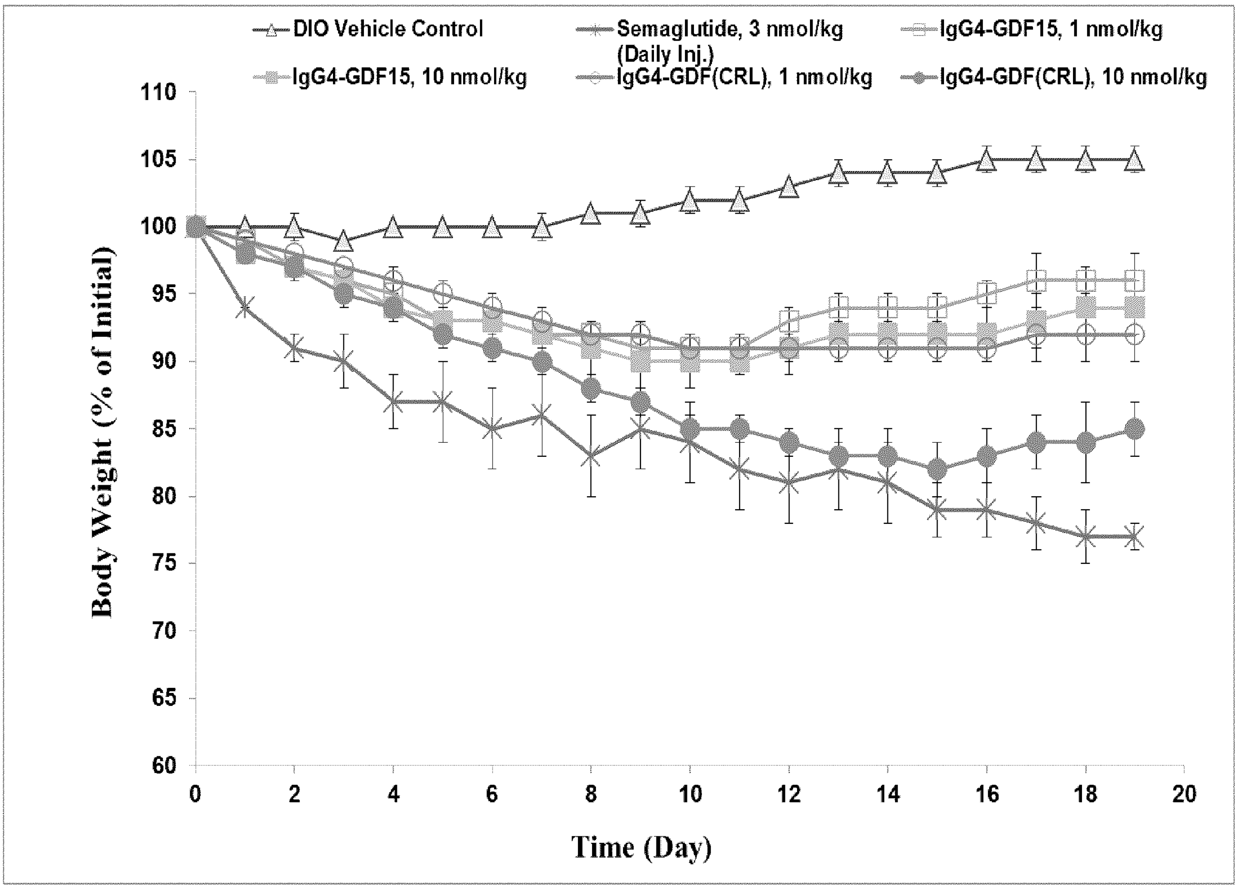
DIO Vehicle Control
Semaglutide, 3 nmol/kg (Daily Inj.)
IgG4-GDF15, 1 nmol/kg
IgG4-GDF15, 10 nmol/kg
IgG4-GDF(CRL), 1 nmol/kg
IgG4-GDF(CRL), 10 nmol/kg
Body Weight (% of Initial)
110
105
100
95
90
85
80
75
70
65
60
0
2
4
6
8
10
12
14
16
18
20
Time (Day)

【FIG. 8】

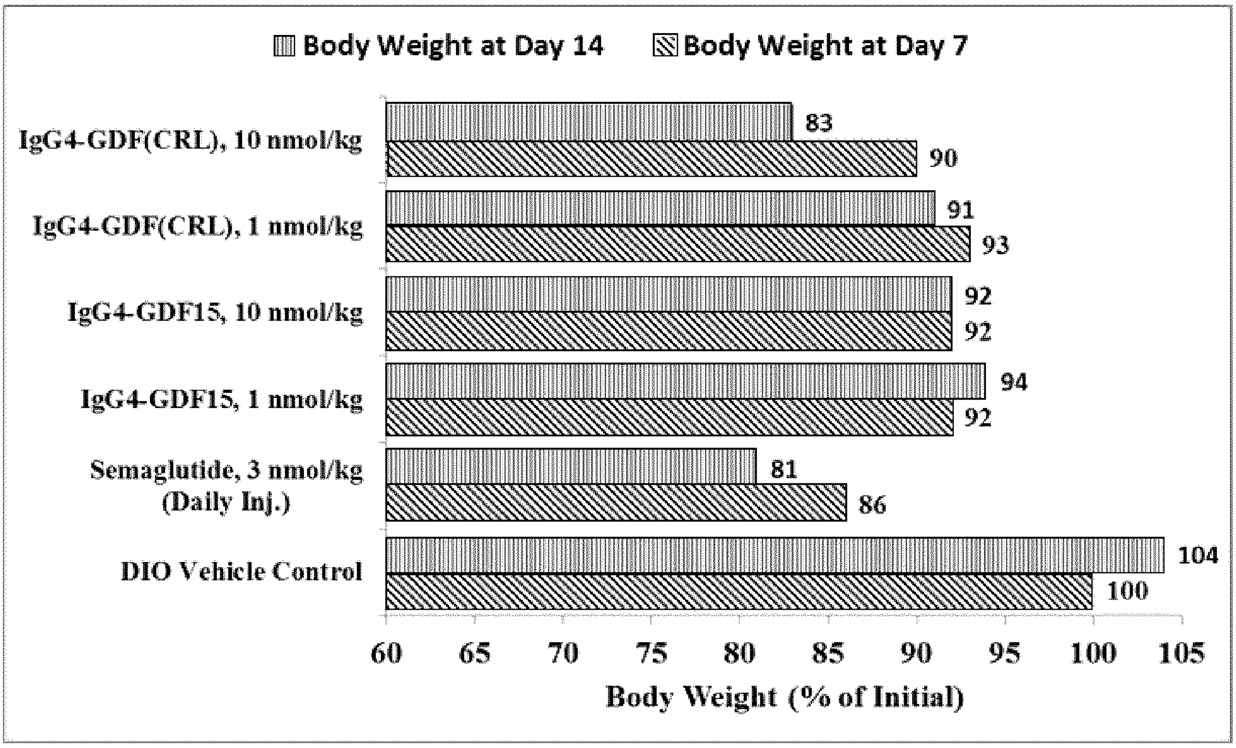
Body Weight at Day 14
Body Weight at Day 7
IgG4-GDF(CRL), 10 nmol/kg
IgG4-GDF(CRL), 1 nmol/kg
IgG4-GDF15, 10 nmol/kg
IgG4-GDF15, 1 nmol/kg
Semaglutide, 3 nmol/kg (Daily Inj.)
DIO Vehicle Control
83
90
91
93
92
92
94
92
81
86
104
100
60
65
70
75
80
85
90
95
100
105
Body Weight (% of Initial)

【FIG. 9】

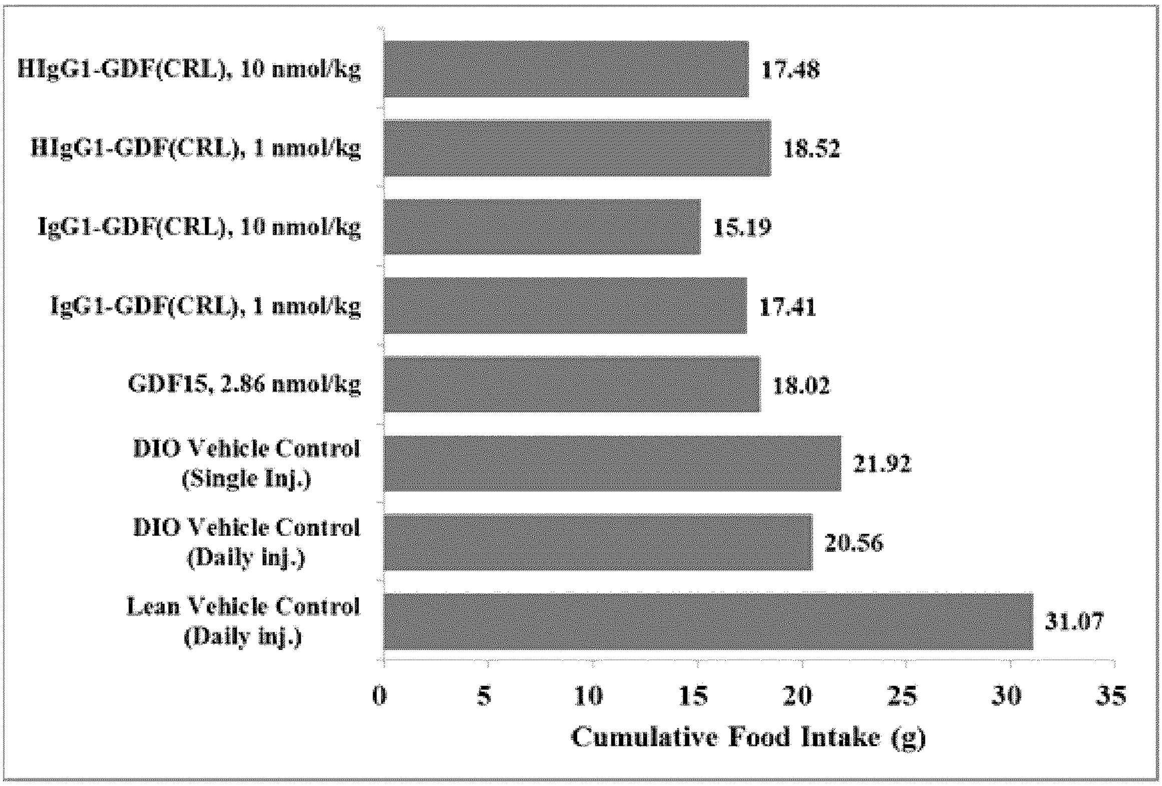
HIgG1-GDF(CRL), 10 nmol/kg
17.48
HIgG1-GDF(CRL), 1 nmol/kg
18.52
IgG1-GDF(CRL), 10 nmol/kg
15.19
IgG1-GDF(CRL), 1 nmol/kg
17.41
GDF15, 2.86 nmol/kg
18.02
DIO Vehicle Control (Single Inj.)
21.92
DIO Vehicle Control (Daily inj.)
20.56
Lean Vehicle Control (Daily inj.)
31.07
0
5
10
15
20
25
30
35
Cumulative Food Intake (g)

【FIG. 10】

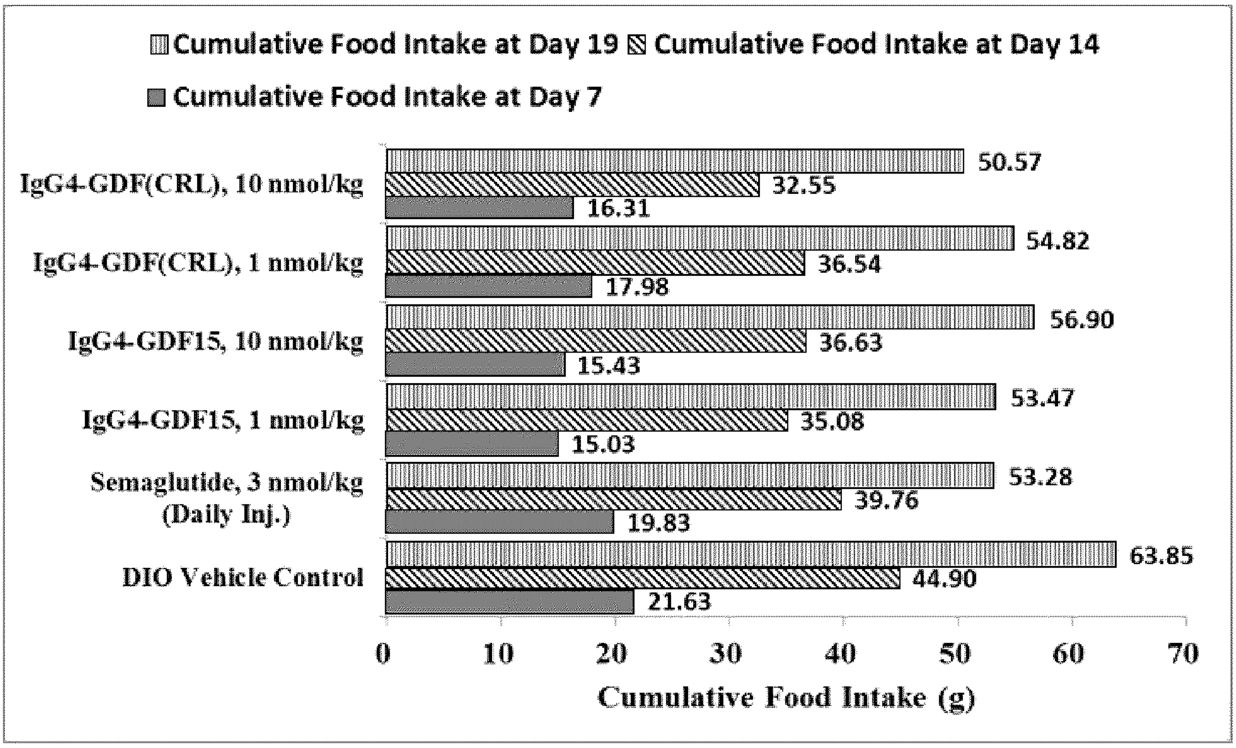
Cumulative Food Intake at Day 19
Cumulative Food Intake at Day 14
Cumulative Food Intake at Day 7
IgG4-GDF(CRL), 10 nmol/kg
50.57
32.55
16.31
IgG4-GDF(CRL), 1 nmol/kg
54.82
36.54
17.98
IgG4-GDF15, 10 nmol/kg
56.90
36.63
15.43
IgG4-GDF15, 1 nmol/kg
53.47
35.08
15.03
Semaglutide, 3 nmol/kg
(Daily Inj.)
53.28
39.76
19.83
DIO Vehicle Control
63.85
44.90
21.63
0
10
20
30
40
50
60
70
Cumulative Food Intake (g)

【FIG. 11】

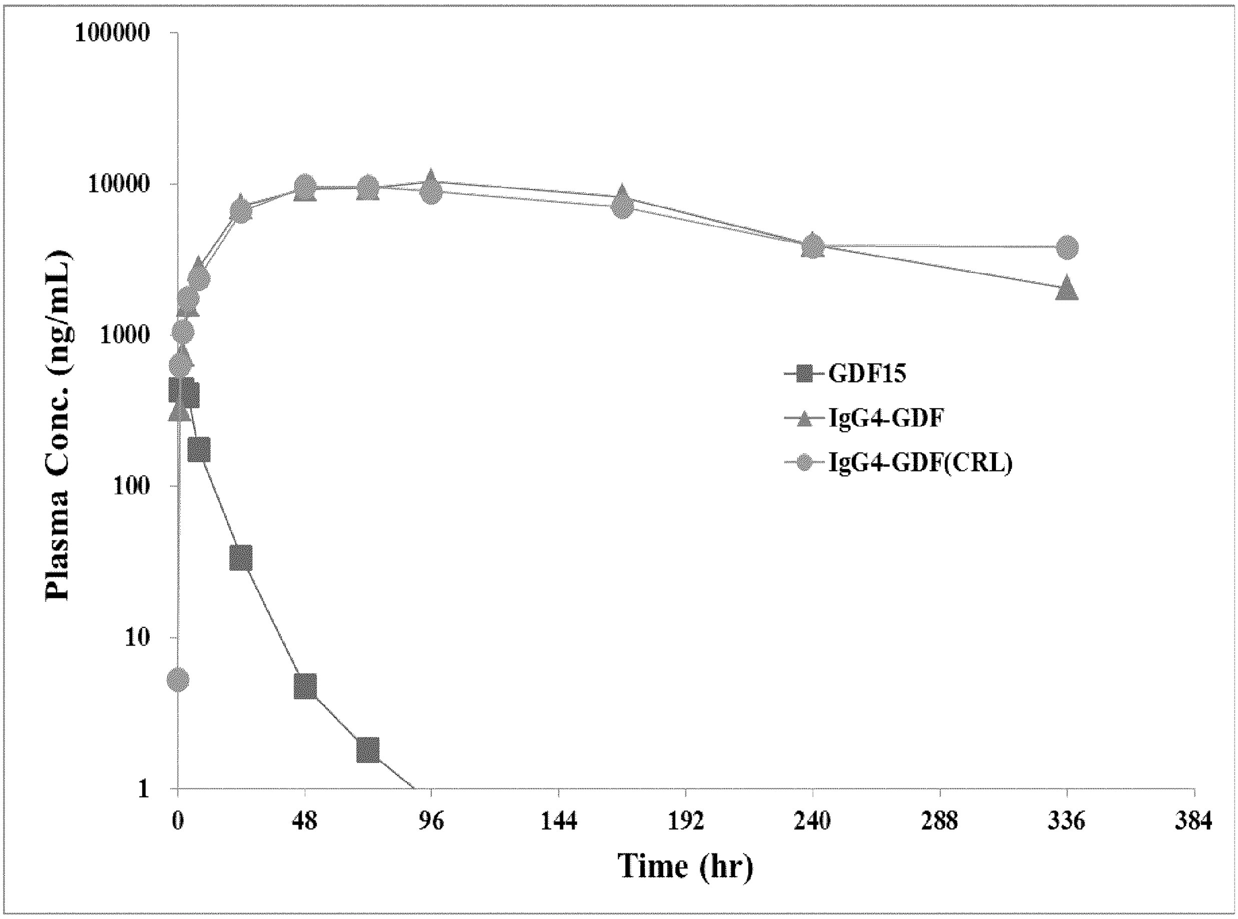
100000
10000
1000
100
10
1
Plasma Conc. (ng/mL)
0
48
96
144
192
240
288
336
384
Time (hr)
GDF15
IgG4-GDF
IgG4-GDF(CRL)

**INTERNATIONAL SEARCH REPORT**

International application No.
**PCT/KR2020/005324**

**A. CLASSIFICATION OF SUBJECT MATTER**

**C07K 14/475**(2006.01)i; ***A61K 38/18***(2006.01)i; ***A61K 47/68***(2017.01)i; ***A61P 1/16***(2006.01)i; ***A61P 3/04***(2006.01)i; ***A61P 3/10***(2006.01)i

According to International Patent Classification (IPC) or to both national classification and IPC

**B. FIELDS SEARCHED**

Minimum documentation searched (classification system followed by classification symbols)

C07K 14/475; A61K 47/50; A61K 47/64; C07K 16/00; A61K 38/18; A61K 47/68; A61P 1/16; A61P 3/04; A61P 3/10

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: GDF15(성장 분화 인자 15; growth differentiation factor-15), 면역글로불린 Fc(immunoglubulin Fc), 융합(fusion), 반감기(half-life), 링커(linker), IgG1, IgG4

**C. DOCUMENTS CONSIDERED TO BE RELEVANT**

| Category* | Citation of document, with indication, where appropriate, of the relevant passages | Relevant to claim No. |
|---|---|---|
| X<br>Y | US 2014-0378665 A1 (AMGEN INC.) 25 December 2014. See abstract; claims 1-29; paragraphs [0138], [0155], [0327]-[0331], [0358], and [0373]-[0375]; and SEQ ID NOs 16, 36, 40, and 129. | 1,5-12,16-21<br>2-4,13-15 |
| Y | EP 3144320 A1 (BRISTOL-MYERS SQUIBB COMPANY) 22 March 2017. See abstract; claims 1-14; and paragraphs [0025], [0036]-[0037], [0044]-[0047], and [0069]. | 2-4,13-15 |
| X<br>Y | US 2016-0168213 A1 (AMGEN INC.) 16 June 2016. See abstract; claims 1-25; and paragraphs [0161]-[0163], [0185], [0964]- [0968], [0995], and [1014]. | 1,5-12,16-21<br>2-4,13-15 |
| A | KR 10-2019-0003746 A (JANSSEN BIOTECH INC.) 09 January 2019. See entire document. | 1-21 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **31 July 2020** | **31 July 2020** |

| Name and mailing address of the ISA/KR | Authorized officer |
|---|---|
| **Korean Intellectual Property Office**<br>**Government Complex Daejeon Building 4, 189, Cheongsa-ro, Seo-gu, Daejeon, 35208, Republic of Korea**<br>**302-701** | |
| Facsimile No. **+82-42-481-8578** | Telephone No. |

Form PCT/ISA/210 (second sheet) (July 2009)

INTERNATIONAL SEARCH REPORT

International application No.
**PCT/KR2020/005324**

**C. DOCUMENTS CONSIDERED TO BE RELEVANT**

| Category* | Citation of document, with indication, where appropriate, of the relevant passages | Relevant to claim No. |
|---|---|---|
| A | KR 10-2017-0020878 A (NOVARTIS AG.) 24 February 2017. See entire document. | 1-21 |

Form PCT/ISA/210 (second sheet) (July 2009)

**INTERNATIONAL SEARCH REPORT**
**Information on patent family members**

International application No.
**PCT/KR2020/005324**

| Patent document cited in search report | Publication date (day/month/year) | Patent family member(s) | Publication date (day/month/year) |
|---|---|---|---|
| US 2014-0378665 A1 | 25 December 2014 | AU 2013-211846 A1 | 14 August 2014 |
| | | BR 112014018575 A2 | 04 July 2017 |
| | | CA 2862745 A1 | 01 August 2013 |
| | | CN 104204218 A | 10 December 2014 |
| | | EA 201491413 A1 | 31 March 2015 |
| | | EP 2807266 A1 | 03 December 2014 |
| | | EP 2807266 B1 | 15 January 2020 |
| | | JP 2015-506373 A | 02 March 2015 |
| | | KR 10-2014-0125803 A | 29 October 2014 |
| | | MX 2014009129 A | 21 November 2014 |
| | | SG 11201404405 A | 28 August 2014 |
| | | US 10336798 B2 | 02 July 2019 |
| | | US 2017-0291929 A1 | 12 October 2017 |
| | | US 2019-0315822 A1 | 17 October 2019 |
| | | US 9714276 B2 | 25 July 2017 |
| | | WO 2013-113008 A1 | 01 August 2013 |
| EP 3144320 A1 | 22 March 2017 | CY 1118546 T1 | 12 July 2017 |
| | | DK 2697257 T3 | 30 January 2017 |
| | | EP 2697257 A2 | 19 February 2014 |
| | | EP 2697257 B1 | 19 October 2016 |
| | | EP 3144320 B1 | 23 May 2018 |
| | | EP 3415528 A2 | 19 December 2018 |
| | | EP 3415528 A3 | 20 February 2019 |
| | | ES 2608835 T3 | 17 April 2017 |
| | | ES 2676499 T3 | 20 July 2018 |
| | | LT 2697257 T | 27 December 2016 |
| | | RS 55609 B1 | 30 June 2017 |
| | | US 10214579 B2 | 26 February 2019 |
| | | US 2014-0113370 A1 | 24 April 2014 |
| | | US 2016-0376346 A1 | 29 December 2016 |
| | | US 2019-0248872 A1 | 15 August 2019 |
| | | US 9469676 B2 | 18 October 2016 |
| | | WO 2012-142515 A2 | 18 October 2012 |
| | | WO 2012-142515 A3 | 06 December 2012 |
| US 2016-0168213 A1 | 16 June 2016 | AR 097181 A1 | 24 February 2016 |
| | | AU 2014-296107 A1 | 04 February 2016 |
| | | AU 2014-296107 B2 | 26 July 2018 |
| | | CA 2918624 A1 | 05 February 2015 |
| | | CL 2016000249 A1 | 22 July 2016 |
| | | CN 105980400 A | 28 September 2016 |
| | | CR 20160097 A | 29 July 2016 |
| | | EA 201690297 A1 | 30 June 2016 |
| | | EP 3027642 A1 | 08 June 2016 |
| | | HK 1225738 A1 | 15 September 2017 |
| | | IL 243749 A | 21 April 2016 |
| | | IL 243749 B | 28 June 2018 |
| | | JP 2016-532690 A | 20 October 2016 |
| | | JP 2019-178132 A | 17 October 2019 |
| | | JP 6509852 B2 | 08 May 2019 |
| | | KR 10-2016-0038896 A | 07 April 2016 |

Form PCT/ISA/210 (patent family annex) (July 2009)

**INTERNATIONAL SEARCH REPORT**
**Information on patent family members**

International application No.
**PCT/KR2020/005324**

| Patent document cited in search report | Publication date (day/month/year) | Patent family member(s) | Publication date (day/month/year) |
|---|---|---|---|
| | | MA 38873 A1 | 29 September 2017 |
| | | MA 38873 B1 | 30 November 2018 |
| | | MX 2016001164 A | 26 July 2016 |
| | | MX 2019012848 A | 28 November 2019 |
| | | PH 12016500208 A1 | 25 April 2016 |
| | | SG 11201600713 A | 26 February 2016 |
| | | TN 2016000035 A1 | 05 July 2017 |
| | | TW 201602345 A | 16 January 2016 |
| | | TW I648401 B | 21 January 2019 |
| | | UA 116665 C2 | 25 April 2018 |
| | | US 2018-0079790 A1 | 22 March 2018 |
| | | US 9862752 B2 | 09 January 2018 |
| | | UY 35686 A | 30 January 2015 |
| | | WO 2015-017710 A1 | 05 February 2015 |
| KR 10-2019-0003746 A | 09 January 2019 | AR 108442 A1 | 22 August 2018 |
| | | AU 2017-263237 A1 | 22 November 2018 |
| | | BR 112018072946 A2 | 19 February 2019 |
| | | CA 3022865 A1 | 16 November 2017 |
| | | CL 2018003148 A1 | 28 December 2018 |
| | | CN 109451726 A | 08 March 2019 |
| | | CO 2018012096 A2 | 22 November 2018 |
| | | CR 20180532 A | 04 March 2019 |
| | | EA 201892561 A1 | 30 April 2019 |
| | | EP 3454832 A1 | 20 March 2019 |
| | | JP 2019-523637 A | 29 August 2019 |
| | | MX 2018013784 A | 28 March 2019 |
| | | NI 201800121 A | 18 February 2019 |
| | | PE 20190352 A1 | 07 March 2019 |
| | | SG 10201912739 A | 27 February 2020 |
| | | SG 11201809700 A | 29 November 2018 |
| | | SV 2018005781 A | 28 March 2019 |
| | | TW 201803892 A | 01 February 2018 |
| | | US 10336812 B2 | 02 July 2019 |
| | | US 2017-0327560 A1 | 16 November 2017 |
| | | US 2019-0292241 A1 | 26 September 2019 |
| | | WO 2017-196647 A1 | 16 November 2017 |
| KR 10-2017-0020878 A | 24 February 2017 | AR 100939 A1 | 09 November 2016 |
| | | AU 2015-280351 A1 | 08 December 2016 |
| | | BR 112016028884 A2 | 31 October 2017 |
| | | CA 2953480 A1 | 30 December 2015 |
| | | CL 2016003281 A1 | 09 June 2017 |
| | | CN 107073130 A | 18 August 2017 |
| | | CR 20160596 A | 08 May 2017 |
| | | EA 201692466 A1 | 28 April 2017 |
| | | EP 3157571 A1 | 26 April 2017 |
| | | GT 201600263 A | 19 July 2019 |
| | | HK 1232160 A1 | 05 January 2018 |
| | | IL 248990 A | 31 January 2017 |
| | | JP 2017-519024 A | 13 July 2017 |
| | | JP 2019-206563 A | 05 December 2019 |

Form PCT/ISA/210 (patent family annex) (July 2009)

**INTERNATIONAL SEARCH REPORT**
**Information on patent family members**

International application No.
**PCT/KR2020/005324**

| Patent document cited in search report | Publication date (day/month/year) | Patent family member(s) | Publication date (day/month/year) |
|---|---|---|---|
| | | MX 2016017395 A | 01 May 2017 |
| | | MX 367520 B | 26 August 2019 |
| | | PE 10952017 A1 | 07 August 2017 |
| | | PE 20171095 A1 | 07 August 2017 |
| | | PH 12016502294 A1 | 13 February 2017 |
| | | SG 11201609704 A | 27 January 2017 |
| | | TN 2016000558 A1 | 04 April 2018 |
| | | TW 201613653 A | 16 April 2016 |
| | | US 10588980 B2 | 17 March 2020 |
| | | US 2016-0030585 A1 | 04 February 2016 |
| | | US 2018-0326079 A1 | 15 November 2018 |
| | | WO 2015-200078 A1 | 30 December 2015 |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 20150125402
- KR 101038126
- KR 101868139

<110> LG CHEM, LTD.

<120> FUSION POLYPEPTIDE COMPRISING Fc REGION OF IMMUNOGLOBULIN AND GDF15

<130> OPP20200894KR

<150> KR 10-2019-0047558
<151> 2019-04-23

<160> 18

<170> KopatentIn 3.0

<210> 1
<211> 112
<212> PRT
<213> Artificial Sequence

<220>
<223> mature GDF15 (wild type)

<400> 1
```
Ala Arg Asn Gly Asp His Cys Pro Leu Gly Pro Gly Arg Cys Cys Arg
  1               5                  10                  15

Leu His Thr Val Arg Ala Ser Leu Glu Asp Leu Gly Trp Ala Asp Trp
             20                  25                  30

Val Leu Ser Pro Arg Glu Val Gln Val Thr Met Cys Ile Gly Ala Cys
         35                  40                  45

Pro Ser Gln Phe Arg Ala Ala Asn Met His Ala Gln Ile Lys Thr Ser
     50                  55                  60

Leu His Arg Leu Lys Pro Asp Thr Val Pro Ala Pro Cys Cys Val Pro
 65                  70                  75                  80

Ala Ser Tyr Asn Pro Met Val Leu Ile Gln Lys Thr Asp Thr Gly Val
                 85                  90                  95

Ser Leu Gln Thr Tyr Asp Asp Leu Leu Ala Lys Asp Cys His Cys Ile
            100                 105                 110
```

<210> 2
<211> 98
<212> PRT
<213> Artificial Sequence

<220>
<223> DeltaN14GDF15

<400> 2
```
Cys Arg Leu His Thr Val Arg Ala Ser Leu Glu Asp Leu Gly Trp Ala
  1               5                  10                  15

Asp Trp Val Leu Ser Pro Arg Glu Val Gln Val Thr Met Cys Ile Gly
             20                  25                  30
```

```
Ala Cys Pro Ser Gln Phe Arg Ala Ala Asn Met His Ala Gln Ile Lys
        35                  40                  45

Thr Ser Leu His Arg Leu Lys Pro Asp Thr Val Pro Ala Pro Cys Cys
    50                  55                  60

Val Pro Ala Ser Tyr Asn Pro Met Val Leu Ile Gln Lys Thr Asp Thr
65                  70                  75                  80

Gly Val Ser Leu Gln Thr Tyr Asp Asp Leu Leu Ala Lys Asp Cys His
                85                  90                  95

Cys Ile



<210>   3
<211>   217
<212>   PRT
<213>   Artificial Sequence

<220>
<223>   human IgG1-Fc (UniProtKB P01857) CH2-CH3


<400>   3
Ala Pro Glu Leu Leu Gly Gly Pro Ser Val Phe Leu Phe Pro Pro Lys
1               5                   10                  15

Pro Lys Asp Thr Leu Met Ile Ser Arg Thr Pro Glu Val Thr Cys Val
            20                  25                  30

Val Val Asp Val Ser His Glu Asp Pro Glu Val Lys Phe Asn Trp Tyr
        35                  40                  45

Val Asp Gly Val Glu Val His Asn Ala Lys Thr Lys Pro Arg Glu Glu
    50                  55                  60

Gln Tyr Asn Ser Thr Tyr Arg Val Val Ser Val Leu Thr Val Leu His
65                  70                  75                  80

Gln Asp Trp Leu Asn Gly Lys Glu Tyr Lys Cys Lys Val Ser Asn Lys
                85                  90                  95

Ala Leu Pro Ala Pro Ile Glu Lys Thr Ile Ser Lys Ala Lys Gly Gln
            100                 105                 110

Pro Arg Glu Pro Gln Val Tyr Thr Leu Pro Pro Ser Arg Asp Glu Leu
        115                 120                 125

Thr Lys Asn Gln Val Ser Leu Thr Cys Leu Val Lys Gly Phe Tyr Pro
    130                 135                 140

Ser Asp Ile Ala Val Glu Trp Glu Ser Asn Gly Gln Pro Glu Asn Asn
145                 150                 155                 160

Tyr Lys Thr Thr Pro Pro Val Leu Asp Ser Asp Gly Ser Phe Phe Leu
                165                 170                 175

Tyr Ser Lys Leu Thr Val Asp Lys Ser Arg Trp Gln Gln Gly Asn Val
            180                 185                 190
```

Phe Ser Cys Ser Val Met His Glu Ala Leu His Asn His Tyr Thr Gln
195 200 205

Lys Ser Leu Ser Leu Ser Pro Gly Xaa
210 215

<210> 4
<211> 10
<212> PRT
<213> Artificial Sequence

<220>
<223> human IgG1-Fc (UniProtKB P01857) Core Hinge

<400> 4
Asp Lys Thr His Thr Cys Pro Pro Cys Pro
1 5 10

<210> 5
<211> 217
<212> PRT
<213> Artificial Sequence

<220>
<223> human IgG4-Fc (UniProtKB P01861) CH2-CH3 (general)

<220>
<221> MOD_RES
<222> (4)
<223> Xaa is Phe or Ala

<220>
<221> MOD_RES
<222> (5)
<223> Xaa is Leu or Ala

<220>
<221> MOD_RES
<222> (217)
<223> Xaa is absent or Lys

<400> 5
Ala Pro Glu Xaa Xaa Gly Gly Pro Ser Val Phe Leu Phe Pro Pro Lys
1 5 10 15

Pro Lys Asp Thr Leu Met Ile Ser Arg Thr Pro Glu Val Thr Cys Val
20 25 30

Val Val Asp Val Ser Gln Glu Asp Pro Glu Val Gln Phe Asn Trp Tyr
35 40 45

Val Asp Gly Val Glu Val His Asn Ala Lys Thr Lys Pro Arg Glu Glu
50 55 60

Gln Phe Asn Ser Thr Tyr Arg Val Val Ser Val Leu Thr Val Leu His
65 70 75 80

```
Gln Asp Trp Leu Asn Gly Lys Glu Tyr Lys Cys Lys Val Ser Asn Lys
                85                  90                  95

Gly Leu Pro Ser Ser Ile Glu Lys Thr Ile Ser Lys Ala Lys Gly Gln
            100                 105                 110

Pro Arg Glu Pro Gln Val Tyr Thr Leu Pro Pro Ser Gln Glu Glu Met
        115                 120                 125

Thr Lys Asn Gln Val Ser Leu Thr Cys Leu Val Lys Gly Phe Tyr Pro
    130                 135                 140

Ser Asp Ile Ala Val Glu Trp Glu Ser Asn Gly Gln Pro Glu Asn Asn
145                 150                 155                 160

Tyr Lys Thr Thr Pro Pro Val Leu Asp Ser Asp Gly Ser Phe Phe Leu
                165                 170                 175

Tyr Ser Arg Leu Thr Val Asp Lys Ser Arg Trp Gln Glu Gly Asn Val
            180                 185                 190

Phe Ser Cys Ser Val Met His Glu Ala Leu His Asn His Tyr Thr Gln
        195                 200                 205

Lys Ser Leu Ser Leu Ser Leu Gly Xaa
    210                 215


<210>  6
<211>  217
<212>  PRT
<213>  Artificial Sequence

<220>
<223>  human IgG4-Fc (UniProtKB P01861) CH2-CH3 (wild-type)


<400>  6
Ala Pro Glu Phe Leu Gly Gly Pro Ser Val Phe Leu Phe Pro Pro Lys
1               5                   10                  15

Pro Lys Asp Thr Leu Met Ile Ser Arg Thr Pro Glu Val Thr Cys Val
            20                  25                  30

Val Val Asp Val Ser Gln Glu Asp Pro Glu Val Gln Phe Asn Trp Tyr
        35                  40                  45

Val Asp Gly Val Glu Val His Asn Ala Lys Thr Lys Pro Arg Glu Glu
    50                  55                  60

Gln Phe Asn Ser Thr Tyr Arg Val Val Ser Val Leu Thr Val Leu His
65                  70                  75                  80

Gln Asp Trp Leu Asn Gly Lys Glu Tyr Lys Cys Lys Val Ser Asn Lys
                85                  90                  95

Gly Leu Pro Ser Ser Ile Glu Lys Thr Ile Ser Lys Ala Lys Gly Gln
            100                 105                 110

Pro Arg Glu Pro Gln Val Tyr Thr Leu Pro Pro Ser Gln Glu Glu Met
        115                 120                 125
```

```
Thr Lys Asn Gln Val Ser Leu Thr Cys Leu Val Lys Gly Phe Tyr Pro
    130                 135                 140

Ser Asp Ile Ala Val Glu Trp Glu Ser Asn Gly Gln Pro Glu Asn Asn
145                 150                 155                 160

Tyr Lys Thr Thr Pro Pro Val Leu Asp Ser Asp Gly Ser Phe Phe Leu
                165                 170                 175

Tyr Ser Arg Leu Thr Val Asp Lys Ser Arg Trp Gln Glu Gly Asn Val
            180                 185                 190

Phe Ser Cys Ser Val Met His Glu Ala Leu His Asn His Tyr Thr Gln
        195                 200                 205

Lys Ser Leu Ser Leu Ser Leu Gly Lys
    210                 215


<210>   7
<211>   216
<212>   PRT
<213>   Artificial Sequence

<220>
<223>   human IgG4-Fc (UniProtKB P01861) CH2-CH3 (mutated)


<400>   7
Ala Pro Glu Ala Ala Gly Gly Pro Ser Val Phe Leu Phe Pro Pro Lys
1               5                   10                  15

Pro Lys Asp Thr Leu Met Ile Ser Arg Thr Pro Glu Val Thr Cys Val
            20                  25                  30

Val Val Asp Val Ser Gln Glu Asp Pro Glu Val Gln Phe Asn Trp Tyr
        35                  40                  45

Val Asp Gly Val Glu Val His Asn Ala Lys Thr Lys Pro Arg Glu Glu
    50                  55                  60

Gln Phe Asn Ser Thr Tyr Arg Val Val Ser Val Leu Thr Val Leu His
65                  70                  75                  80

Gln Asp Trp Leu Asn Gly Lys Glu Tyr Lys Cys Lys Val Ser Asn Lys
                85                  90                  95

Gly Leu Pro Ser Ser Ile Glu Lys Thr Ile Ser Lys Ala Lys Gly Gln
            100                 105                 110

Pro Arg Glu Pro Gln Val Tyr Thr Leu Pro Pro Ser Gln Glu Glu Met
        115                 120                 125

Thr Lys Asn Gln Val Ser Leu Thr Cys Leu Val Lys Gly Phe Tyr Pro
    130                 135                 140

Ser Asp Ile Ala Val Glu Trp Glu Ser Asn Gly Gln Pro Glu Asn Asn
145                 150                 155                 160

Tyr Lys Thr Thr Pro Pro Val Leu Asp Ser Asp Gly Ser Phe Phe Leu
                165                 170                 175

Tyr Ser Arg Leu Thr Val Asp Lys Ser Arg Trp Gln Glu Gly Asn Val
```

180 185 190

Phe Ser Cys Ser Val Met His Glu Ala Leu His Asn His Tyr Thr Gln
195 200 205

Lys Ser Leu Ser Leu Ser Leu Gly
210 215

<210> 8
<211> 216
<212> PRT
<213> Artificial Sequence

<220>
<223> human IgG4-Fc (UniProtKB P01861) CH2-CH3 (mutated)

<400> 8
Ala Pro Glu Ala Leu Gly Gly Pro Ser Val Phe Leu Phe Pro Pro Lys
1 5 10 15

Pro Lys Asp Thr Leu Met Ile Ser Arg Thr Pro Glu Val Thr Cys Val
20 25 30

Val Val Asp Val Ser Gln Glu Asp Pro Glu Val Gln Phe Asn Trp Tyr
35 40 45

Val Asp Gly Val Glu Val His Asn Ala Lys Thr Lys Pro Arg Glu Glu
50 55 60

Gln Phe Asn Ser Thr Tyr Arg Val Val Ser Val Leu Thr Val Leu His
65 70 75 80

Gln Asp Trp Leu Asn Gly Lys Glu Tyr Lys Cys Lys Val Ser Asn Lys
85 90 95

Gly Leu Pro Ser Ser Ile Glu Lys Thr Ile Ser Lys Ala Lys Gly Gln
100 105 110

Pro Arg Glu Pro Gln Val Tyr Thr Leu Pro Pro Ser Gln Glu Glu Met
115 120 125

Thr Lys Asn Gln Val Ser Leu Thr Cys Leu Val Lys Gly Phe Tyr Pro
130 135 140

Ser Asp Ile Ala Val Glu Trp Glu Ser Asn Gly Gln Pro Glu Asn Asn
145 150 155 160

Tyr Lys Thr Thr Pro Pro Val Leu Asp Ser Asp Gly Ser Phe Phe Leu
165 170 175

Tyr Ser Arg Leu Thr Val Asp Lys Ser Arg Trp Gln Glu Gly Asn Val
180 185 190

Phe Ser Cys Ser Val Met His Glu Ala Leu His Asn His Tyr Thr Gln
195 200 205

Lys Ser Leu Ser Leu Ser Leu Gly
210 215

<210> 9

<211> 216
<212> PRT
<213> Artificial Sequence

<220>
<223> human IgG4-Fc (UniProtKB P01861) CH2-CH3 (mutated)

<400> 9

```
Ala Pro Glu Phe Ala Gly Gly Pro Ser Val Phe Leu Phe Pro Pro Lys
1               5                   10                  15

Pro Lys Asp Thr Leu Met Ile Ser Arg Thr Pro Glu Val Thr Cys Val
            20                  25                  30

Val Val Asp Val Ser Gln Glu Asp Pro Glu Val Gln Phe Asn Trp Tyr
        35                  40                  45

Val Asp Gly Val Glu Val His Asn Ala Lys Thr Lys Pro Arg Glu Glu
    50                  55                  60

Gln Phe Asn Ser Thr Tyr Arg Val Val Ser Val Leu Thr Val Leu His
65                  70                  75                  80

Gln Asp Trp Leu Asn Gly Lys Glu Tyr Lys Cys Lys Val Ser Asn Lys
                85                  90                  95

Gly Leu Pro Ser Ser Ile Glu Lys Thr Ile Ser Lys Ala Lys Gly Gln
            100                 105                 110

Pro Arg Glu Pro Gln Val Tyr Thr Leu Pro Pro Ser Gln Glu Glu Met
        115                 120                 125

Thr Lys Asn Gln Val Ser Leu Thr Cys Leu Val Lys Gly Phe Tyr Pro
    130                 135                 140

Ser Asp Ile Ala Val Glu Trp Glu Ser Asn Gly Gln Pro Glu Asn Asn
145                 150                 155                 160

Tyr Lys Thr Thr Pro Pro Val Leu Asp Ser Asp Gly Ser Phe Phe Leu
                165                 170                 175

Tyr Ser Arg Leu Thr Val Asp Lys Ser Arg Trp Gln Glu Gly Asn Val
            180                 185                 190

Phe Ser Cys Ser Val Met His Glu Ala Leu His Asn His Tyr Thr Gln
        195                 200                 205

Lys Ser Leu Ser Leu Ser Leu Gly
    210                 215
```

<210> 10
<211> 12
<212> PRT
<213> Artificial Sequence

<220>
<223> human IgG4-Fc (UniProtKB P01861) Hinge (general)

<220>
<221> MOD_RES

<222> (10)
<223> Xaa is Ser or Pro

<400> 10
Glu Ser Lys Tyr Gly Pro Pro Cys Pro Xaa Cys Pro
1 5 10

<210> 11
<211> 12
<212> PRT
<213> Artificial Sequence

<220>
<223> human IgG4-Fc (UniProtKB P01861) Hinge (wild-type)

<400> 11
Glu Ser Lys Tyr Gly Pro Pro Cys Pro Ser Cys Pro
1 5 10

<210> 12
<211> 12
<212> PRT
<213> Artificial Sequence

<220>
<223> human IgG4-Fc (UniProtKB P01861) Hinge (mutated)

<400> 12
Glu Ser Lys Tyr Gly Pro Pro Cys Pro Pro Cys Pro
1 5 10

<210> 13
<211> 5
<212> PRT
<213> Artificial Sequence

<220>
<223> peptide linker

<400> 13
Gly Gly Gly Gly Ser
1 5

<210> 14
<211> 339
<212> DNA
<213> Artificial Sequence

<220>
<223> GDF15 coding gene

<400> 14
gcccggaacg gcgaccactg ccccctgggg cccggacggt gctgccggct gcacaccgtg 60

cgggcctccc tggaggacct gggctgggcc gactgggtgc tgtccccaag ggaggtgcaa 120

gtgaccatgt gcatcggcgc ctgcccatct cagttccggg ccgccaacat gcacgctcag 180

atcaagacca gcctgcaccg gctgaagccc gacaccgtgc ccgccccctg ctgcgtgccc 240

gcctcctaca accccatggt gctgattcag aagaccgaca ccggcgtgag cctgcagacc 300

tacgacgacc tgctggccaa ggactgccac tgcatctaa 339

<210> 15
<211> 678
<212> DNA
<213> Artificial Sequence

<220>
<223> IgG1-Fc coding gene

<400> 15
gacaaaactc acacatgccc accgtgccca gcacctgaac tcctgggggg accgtcagtc 60

ttcctcttcc ccccaaaacc caaggacacc ctcatgatct cccggacccc tgaggtcaca 120

tgcgtggtgg tggacgtgag ccacgaagac cctgaggtca agttcaactg gtacgtggac 180

ggcgtggagg tgcataatgc caagacaaag ccgcgggagg agcagtacaa cagcacgtac 240

cgtgtggtca gcgtcctcac cgtcctgcac caggactggc tgaatggcaa ggagtacaag 300

tgcaaggtct ccaacaaagc cctcccagcc cccatcgaga aaaccatctc caaagccaaa 360

gggcagcccc gagaaccaca ggtgtatacc ctgcccccat cccgggatga gctgaccaag 420

aaccaggtca gcctgacctg cctggtcaaa ggcttctatc ccagcgacat cgccgtggag 480

tgggagagca atgggcagcc ggagaacaac tacaagacca cgcctcccgt gctggactcc 540

gacggctcct tcttcctcta cagcaagctc accgtggaca agagcaggtg gcagcagggg 600

aacgtcttct catgctccgt gatgcatgag gctctgcaca accactacac gcagaagagc 660

ctctccctgt ctccgggt 678

<210> 16
<211> 684
<212> DNA
<213> Artificial Sequence

<220>
<223> IgG4-Fc coding gene

<400> 16
gagtccaaat atggtccccc atgcccaccc tgcccagcac ctgaggccgc cgggggaccg 60

tcagtcttcc tcttcccccc aaaacccaag gacaccctca tgatctcccg gacccctgag 120

gtcacgtgcg tggtggtgga cgtgtcccag gaggaccccg aggtgcagtt caactggtac 180

gtggacggcg tggaggtgca caacgccaag accaagcccc gggaggagca gttcaactcc 240

```
acctaccggg tggtgtccgt gctgaccgtg ctgcaccagg actggctgaa cggcaaggag      300

tacaagtgca aggtgtccaa caagggcctg ccctcctcca tcgagaagac catctccaag      360

gccaagggcc agccccggga gccccaggtg tacaccctgc ccccctccca ggaggagatg      420

accaagaacc aggtgtccct gacctgcctg gtgaagggct tctacccctc cgacatcgcc      480

gtggagtggg agtccaacgg ccagcccgag aacaactaca agaccacccc ccccgtgctg      540

gactccgacg gctccttctt cctgtactcc cggctgaccg tggacaagtc ccggtggcag      600

gagggcaacg tgttctcctg ctccgtgatg cacgaggccc tgcacaacca ctacacccag      660

aagtccctgt ccctgtccct gggc                                             684


<210>  17
<211>  22
<212>  PRT
<213>  Artificial Sequence

<220>
<223>  Signal Peptide


<400>  17
Met His Arg Pro Glu Ala Met Leu Leu Leu Leu Thr Leu Ala Leu Leu
1               5                   10                  15

Gly Gly Pro Thr Trp Ala
            20


<210>  18
<211>  20
<212>  PRT
<213>  Artificial Sequence

<220>
<223>  peptide linker


<400>  18
Gly Gly Gly Gly Ser Gly Gly Gly Gly Ser Gly Gly Gly Gly Ser Gly
1               5                   10                  15

Gly Gly Gly Ser
            20
```